# EUROPÄISCHE PATENTSCHRIFT

(11) **EP 0 397 060 B1**
(45) Veröffentlichungstag und Bekanntmachung des Hinweises auf die Patenterteilung: **21.11.2001**
(21) Anmeldenummer: 90108468.1
(22) Anmeldetag: 04.05.1990
(51) Int. Cl.: C07D 403/14, C07D 491/04, A61K 31/40

(54) **Maleinimid-Derivate und deren Verwendung als Arzneimittel**
Maleinimide derivatives and their use as medicines
Dérivés de maléinimide et leur utilisation comme médicament

(30) Priorität: 05.05.1989 DE 3914764; 27.12.1989 DE 3942991
(43) Veröffentlichungstag der Anmeldung: 14.11.1990
(73) Patentinhaber: GÖDECKE AKTIENGESELLSCHAFT, D-10587 Berlin (DE)
(72) Erfinder: Barth, Hubert, Dr., D-7830 Emmendingen (DE); Hartenstein, Johannes, Dr., D-7801 Stegen-Wittental (DE); Rudolph, Claus, Dr., D-7801 Vörstetten (DE); Schächtele, Christoph, Dr., D-7800 Freiburg (DE); Betche, Hans-Jürgen, Dr., D-7801 Vörstetten (DE); Osswald, Hartmut, Dr., D-7830 Emmendingen (DE); Reck, Reinhard, Dr., D-7831 Sexau (DE)

(56) Entgegenhaltungen:
- EP-A- 0 269 025
- EP-A- 0 328 026
- TETRAHEDRON LETTERS, Band 28, Nr. 38, 1987, Seiten 4441-4444, Oxford, GB; J. BERGMAN et al.: "Coupling of indoleacetic acid trianion or methyl indoleacetic acid dianion. A biomimetic approach to indolocarbazole alkaloids"
- FEBS LETTERS, Band 259, Nr. 1, 18. Dezember 1989, Seiten 61-63, Amsterdam, NL; P.D. DAVIS et al.: "Potent selective inhibitors of protein kinase C"

## Beschreibung

Die Erfindung betrifft in den Beispielen genan definierte, Bis-(1H-indol-3-yl)maleinimid-Derivate der allgemeinen Formel I, 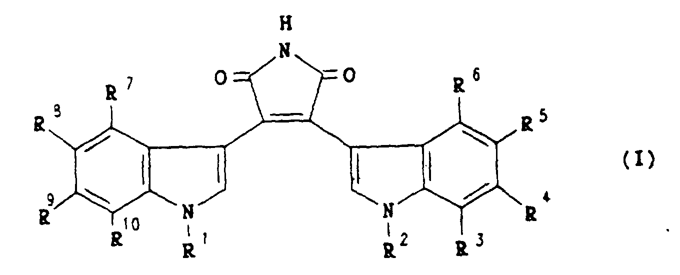 und deren pharmakologisch unbedenkliche Salze sowie Arzneimittel mit einem Gehalt an mindestens einer der Verbindungen I wie in den Ansführungs beispielen genem definiert, sowie deren, zur Herstellung von Arzneimitteln für die Prävention oder Behandlung von Krankheiten, bei denen die Hemmung der Proteinkinase C von Bedeutung ist.

Ein weiterer Gegenstand der Erfindung sind Arzneimittel enthaltend neben üblichen Hilfs- und Zusatzstoffen Verbindungen der allgemeinen Formel I wie in den Beispielen genan definiert.

Die Darstellung der Verbindungen der allgemeinen Formel I gemäß Anspruch 1 erfolgt in Analogie zu den in der Literatur beschriebenen Verfahren (Tetrahedron 1988, 44, 2887; Tetrahedron Lett. 1985, 4015; EP-A-0 269 025) oder durch geeignete Abwandlungen dieser Verfahren.

Diese Verfahren sind dadurch gekennzeichnet, daß man entweder
A) ein Dibrommaleinimid der allgemeinen Formel II, 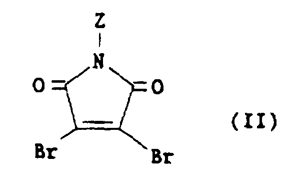 in welcher Z eine geeignete abspaltbare Schutzgruppe ist, mit einem Indol-Grignardreagenz der allgemeinen Formel A, 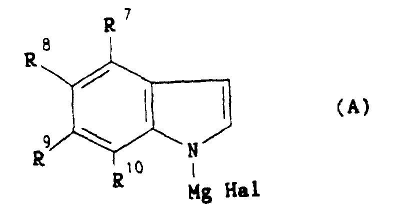 in welcher R⁷, R⁸, R⁹ und R¹⁰ die in den Einzelverbindungen genannte Bedeutung haben, nicht jedoch Hydroxy oder Acyloxy sind, nach an sich bekannten Methoden umsetzt, und das erhaltene Produkt der allgemeinen Formel III, 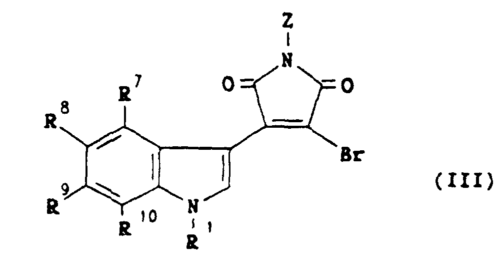 bei der R¹ Wasserstoff bedeutet, gegebenenfalls am Indolstickstoff mit einem Alkylierungsmittel R¹-X, wobei R¹ mit Ausnahme von Wasserstoff die in den Einzelverbindungen genannten Bedeutungen hat, nicht jedoch funktionelle Gruppen enthält, die die nachfolgenden Umsetzungen stören würden, und X für eine leicht austretende Gruppe, wie z.B. Chlor oder Brom, steht, in an sich bekannter Weise alkyliert, wobei ein Produkt der allgemeinen Formel III erhalten wird, bei dem R¹ verschieden von Wasserstoff ist, und anschließend Produkt III mit einem Indolgrignardreagenz der allgemeinen Formel (B), 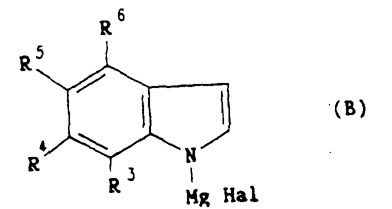 in welcher R³, R⁴, R⁵ und R⁶ die in den Einzelverbindungen genannte Bedeutung haben, nicht jedoch Hydroxy oder Acyloxy sind, umsetzt und gegebenenfalls anschließend mit einem Alkylierungsmittel der allgemeinen Formel R²-X, in der R² mit Ausnahme von Wasserstoff die in den Einzelverbindungen genannten Bedeutungen hat, nicht jedoch funktionelle Gruppen enthält, die die nachfolgenden Umsetzungen stören würden und X die oben genannte Bedeutung hat, alkyliert zu einem Produkt der allgemeinen Formel IV, 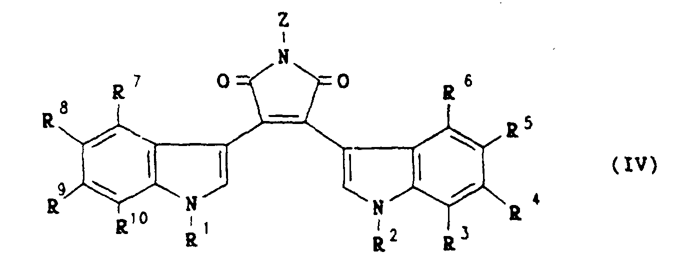 wonach man die substituierte Imidgruppe in die unsubstituierte Imidgruppe der Verbindung der allgemeinen Formel I überführt, oder
B) ein Produkt der allgemeinen Formel V 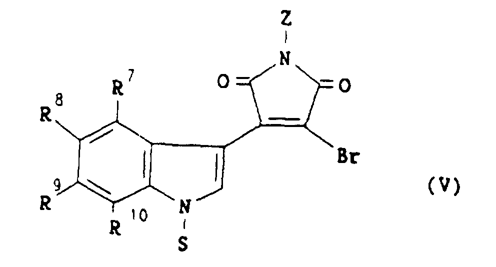 bei der S und Z eine geeignete abspaltbare Schutzgruppe bedeuten, mit einem Indolgrignardreagenz der allgemeinen Formel (B) 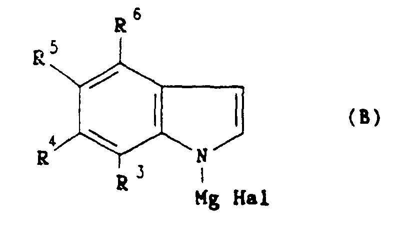 in welcher R³, R⁴, R⁵ und R⁶ die in den Einzelverbindungen genannte Bedeutung haben, nicht jedoch Hydroxy oder Acyloxy sind, umsetzt und gegebenenfalls anschließend mit einem Alkylierungsmittel der allgemeinen Formel R²-X, in der R² mit Ausnahme von Wasserstoff die in den Einzelverbindungen genannten Bedeutungen hat, nicht jedoch funktionelle Gruppen enthält, die die nachfolgenden Umsetzungen stören würden und X die oben genannte Bedeutung hat, alkyliert zu einem Produkt der allgemeinen Formel VI 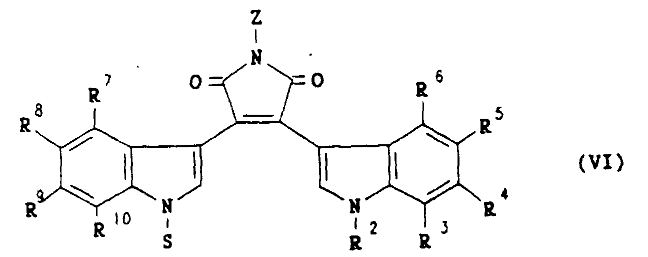 wonach man die geschützte Indolgruppe in die freie Indolgruppe und die substituierte Imidgruppe in die unsubstituierte Imidgruppe der Verbindung der allgemeinen Formel I überführt, wobei R¹ für Wasserstoff steht, und R² bis R¹⁰ die in den Einzelverbindungen genannte Bedeutung haben, oder
C) ein Dibrommaleinimid der allgemeinen Formel II 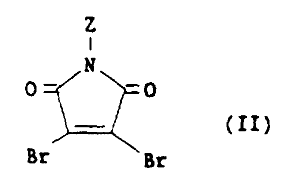 in welcher Z eine geeignete abspaltbare Schutzgruppe ist, mit einem Überschuß an Indolgrignardreagenz der allgemeinen Formel (A) 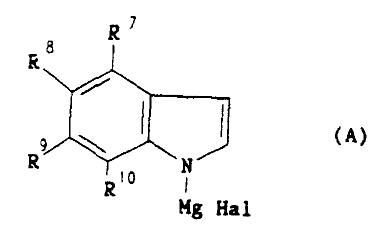 in welcher R⁷, R⁸, R⁹ und R¹⁰ die in den Einzelverbingungen die genannte Bedeutung haben, nicht jedoch Hydroxy oder Acyloxy sind, umsetzt und das erhaltene Produkt der allgemeinen Formel VII 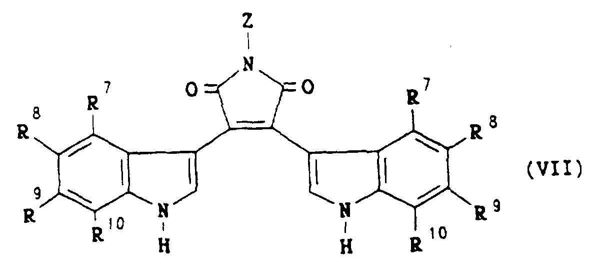 gegebenenfalls am Indolstickstoff mit einem Alkylierungsmittel R¹-X, wobei R¹ mit Ausnahme von Wasserstoff die in den Einzelverbindungen genannten Bedeutungen hat, nicht jedoch funktionelle Gruppen enthält, die die nachfolgenden Umsetzungen stören würden und X für eine leicht austretende Gruppe, wie z.B. Chlor oder Brom, steht, alkyliert, wobei ein Produkt der allgemeinen Formel VIII 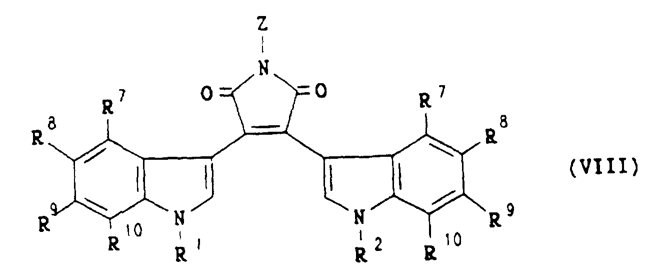 erhalten wird, bei der R¹ verschieden von Wasserstoff und R² gleich Wasserstoff und R⁷ bis R¹⁰ eine der in den Einzelverbindungen genannten Bedeutungen besitzen, nicht jedoch für Hydroxy oder Acyloxy stehen, wonach man die substituierte Imidgruppe in die unsubstituierte Imidgruppe der Verbindung der allgemeinen Formel I überführt.

Produkte der allgemeinen Formel VIII, in denen R¹ verschieden von Wasserstoff ist und R² gleich Wasserstoff ist, können mit einem Alkylierungsmittel R²-X, wobei R² mit Ausnahme von Wasserstoff die in den Einzelverbindungen genannten Bedeutungen hat und von R¹ verschieden ist und X für eine leicht austretende Gruppe, wie z.B. Chlor oder Brom, steht, zu einem Produkt der allgemeinen Formel VIII umgesetzt werden wobei R¹ ungleich R² und beide Reste verschieden von Wasserstoff sind.

Nach Überführung der substituierten Imidgruppe in VII bzw. VIII in die freie Imidgruppe gelangt man zu Verbindungen der allgemeinen Formel I, in der R¹ bis R¹⁰ die in den Einzelverbindungen genannten Bedeutungen besitzen und R³ gleich R¹⁰, R⁴ gleich R⁹, R⁵ gleich R⁸ und R⁶ gleich R⁷ sind.

Symmetrisch substituierte Bisindolylmaleinimide der allgemeinen Formel I, bei der R¹ und R² Wasserstoff sind, und R³ gleich R¹⁰, R⁴ gleich R⁹, R⁵ gleich R⁸, R⁶ gleich R⁷ sind und die in den Einzelverbindungen angegebene Bedeutungen haben, nicht jedoch Hydroxyl oder Acyloxy sind, können auch durch Umsetzung von Dibrommaleinimid mit einem Überschuß an Indolgrignardreagenz der allgemeinen Formel A erhalten werden.

Die in den Verfahren A bis C beschriebene Überführung der substituierten Imide in die freien Imide kann in Analogie zu den in der Literatur beschriebenen Verfahren erfolgen. Bedeutet Z z.B. Methyl, so wird durch Umsetzung mit einer Lösung von Kaliumhydroxid in Wasser/Methanol und anschließendem Ansäuern zunächst das entsprechende cyclische Anhydrid hergestellt und dieses durch Erhitzen mit Ammoniumacetat oder ethanolischer Ammoniaklösung in das freie Imid übergeführt. (Tetrahedron 44, 1988, 2887). Bedeutet Z z.B. Benzyloxymethyl, so wird die Überführung ins freie Imid durch Hydrierung über Palladium/Kohle durchgeführt. (Tetrahedron Lett. 26, 1985, 4015).

Als Schutzgruppe S in Verfahren B eignen sich besonders die tert.-Butoxycarbonylgruppe (BOC-Gruppe), der p-Toluolsulfonsäurerest (Tosyl-Rest) und die Trimethylsilylethoxymethylgruppe (SEM-Gruppe). Die BOC-Gruppe und der Tosylrest werden bei der Überführung des substituierten Imids ins freie Imid abgespalten, die SEM-Gruppe kann mit Tetra-n-butylammoniumfluorid leicht abgespalten werden (J. Org. Chem. 49, 1984, 205).

Zur Durchführung der Alkylierungen nach Verfahren A bis C mit Verbindungen R¹-X bzw. R²-X besonders geeignete Basen sind Hydride, Carbonate, Amide, Hydroxide, Oxide oder Alkoxide der Alkali- oder Erdalkalimetalle oder lithiumorganische Verbindungen. Als Lösungsmittel eignen sich insbesondere dipolare aprotische Lösungsmittel wie Aceton, Dimethylsulfoxid, Dimethylformamid oder Tetrahydrofuran. Die Glycosidierung erfolgt unter Verwendung geeigneter O-acylierter Glycosylhalogenide.

Verbindungen der allgemeinen Formel I, bei denen R¹ und/oder R² einen Alkoxycarbonylalkylrest bedeuten, können nach Verfahren A bis C, ausgehend von N-Benzyloxymethyldibrommaleinimid und unter Verwendung geeigneter Alkoxycarbonylalkylhalogenide hergestellt werden. Die Alkylierung erfolgt in diesem Falle nach Durchführung der Reaktionen mit dem Indolgrignardreagenz. Durch Reaktion an der Alkoxycarbonylgruppe mit geeigneten Aminen können hieraus nach an sich bekannten Methoden die entsprechenden Amide hergestellt werden.

Verbindungen der allgemeinen Formel I, bei denen R¹ und/oder R² einen Halogenalkylrest bedeuten, werden aus den entsprechenden Hydroxyalkylverbindungen nach an sich bekannten Methoden zur Überführung eines Alkohols in ein Halogenid hergestellt.

Verbindungen der allgemeinen Formel I, bei denen R¹ und/oder R² einen Amidinothioalkylrest bedeuten, werden aus den entsprechenden Halogenalkylverbindungen durch Umsetzung mit Thioharnstoff erhalten.

Verbindungen der allgemeinen Formel I, bei denen R¹ und/oder R² eine Nitroguanidinoalkylgruppe bedeuten, werden aus den entsprechenden Aminoalkylverbindungen durch Umsetzung mit 1-Nitroguanyl-3,4-dimethylpyrazol erhalten.

Verbindungen der allgemeinen Formel I, bei denen R¹ und/oder R² eine Isothiocyanatoalkylgruppe bedeuten, werden aus den entsprechenden Aminoalkylverbindungen durch Umsetzung mit Thiocarbonyldiimidazol erhalten.

Verbindungen der allgemeinen Formel I, bei denen R¹ und/oder R² gleich Acyl bedeutet, werden aus den Verbindungen der allgemeinen Formel I mit R¹ und/oder R² gleich Wasserstoff durch Acylierung unter Verwendung eines geeigneten Acylhalogenids oder Anhydrids in einem geeigneten Lösungsmittel wie z.B. Pyridin hergestellt.

Verbindungen der allgemeine Formel I, bei denen R¹ und/oder R² einen Epoxyalkylrest bedeutet, werden aus Verbindungen der allgemeinen Formel I, bei der R¹ und/oder R² gleich Wasserstoff sind und R³ bis R¹⁰ die in den Einzelverbindungen angegebene Bedeutung besitzen, nicht jedoch für Hydroxyl stehen, durch Umsetzung mit einem geeigneten Halogenepoxyalkan hergestellt. Erfolgt die Umsetzung von Halogenepoxyalkanen mit Verbindungen der allgemeinen Formel VI oder VII und steht Z für Methyl, so wird der Epoxidring bei der Überführung des substituierten Imids ins freie Imid unter Bildung von vicinalen Diolen geöffnet und man erhält Verbindungen der allgemeinen Formel I, bei denen R¹ und/oder R² für einen Dihydroxyalkylrest steht. Der Epoxidring kann auch vor der Abspaltung der Schutzgruppe Z mit Aminen geöffnet werden. Nach Überführung der substituierten Imidgruppe in die freie Imidgruppe erhält man Verbindungen der allgemeinen Formel I, in welcher R¹ und/oder R² für einen Aminohydroxyalkylrest stehen.

Verbindungen der allgemeinen Formel I, in welcher einer oder bis zu vier der Reste R³ bis R¹⁰ für eine Hydroxygruppe stehen, können in an sich bekannter Weise durch Etherspaltung von Verbindungen der allgemeinen Formel I, in welcher einer oder mehrere der Reste R³ bis R¹⁰ für eine C1-C4-Alkoxygruppe stehen, hergestellt werden.

Verbindungen der allgemeinen Formel I, in welcher einer oder bis zu vier der Reste R³ bis R¹⁰ für eine unsubstituierte oder substituierte Aminoalkoxygruppe bzw. Acyloxygruppe stehen, können in an sich bekannter Weise durch Aminoalkylierung bzw. Acylierung von Verbindungen der allgemeinen Formel I, in welcher einer oder bis zu vier der Reste R³ bis R¹⁰ für eine Hydroxygruppe stehen, hergestellt werden.

Die Verbindungen gemäß Anspruch 1 können auch nach den in der EP-A-0 328 026 genannten Verfahren hergestellt werden.

Verbindungen der allgemeinen Formel I, die ein chirales Zentrum aufweisen, können als Stereoisomerengemische oder in Form der Enantiomeren verwendet werden. Die Enantiomeren können nach den für optische Trennungen von Stereoisomeren verwendeten üblichen Verfahren erhalten werden.

Verbindungen der allgemeinen Formel I können gegebenenfalls als Regioisomerengemische anfallen, die nach allgemein bekannten Verfahren in die Einzelverbindungen aufgetrennt werden können.

Gegenstand der vorliegenden Erfindung sind daher auch die Enantiomeren der Stereoisomerengemische sowie die Einzelverbindungen der Regioisomerengemische.

Basische Verbindungen der allgemeinen Formel I, welche ein basisches Zentrum an mindestens einem der Reste R¹ bis R¹⁰ aufweisen, werden zum Zwecke der Reinigung und aus galenischen Gründen bevorzugt in kristalline, pharmakologisch verträgliche Salze übergeführt. Die Salze werden in üblicher Weise durch Neutralisation der Basen mit entsprechenden anorganischen oder organischen Säuren erhalten. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Fumarsäure, Oxalsäure oder Bernsteinsäure in Frage. Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol oder 2-Propanol oder einem niederen Keton wie Aceton oder 2-Butanon oder einem Ether wie Diethylether, Diisopropylether, Tetrahydrofuran oder Dioxan, erhalten.

Die erfindungsgemäßen Verbindungen sind potente Inhibitoren der Proteinkinase C. Überraschernderweise wirken sie selektiv, denn man benötigt sehr viel höhere Konzentrationenen an erfindungsgemäßen Verbindungen, um andere Kinasen wie z.B. die A-Kinase, die G-Kinase oder die MLC-Kinase zu hemmen. Dies trifft insbesondere für Verbindungen der allgemeinen Formel I zu, bei denen der eine Indolrest am Indolstickstoff unsubstituiert ist, während der andere Indolrest am Indolstickstoff und im Benzoteil geeignet substituiert ist. So zeigt z.B. die Verbindung von Beispiel 1 im Enzym-Assay der Proteinkinase C eine 50%ige Inhibierung bei einer Konzentration von 0,14 *µ*mol/Liter, während die IC50-Werte gegenüber der A-Kinase, der G-Kinase und der MLC-Kinase 77 *µ*mol/Liter, 15 *µ*mol/liter bzw. 4,5 *µ*mol/liter betragen.

Die Proteinkinase C spielt für die intracelluläre Signaltransduktion eine wichtige Schlüsselrolle und ist eng mit der Regulation von kontraktilen, sekretorischen und proliferativen Prozessen verknüpft. Aufgrund dieser Eigenschaften können die erfindungsgemäßen Verbindungen zur Behandlung von Herz- und Gefäßkrankheiten wie Thrombosen, Arteriosklerose, Hypertension, von Entzündungsprozessen, Allergien, Krebs und bestimmten degenerativen Schäden des Zentralnervensystems sowie von Krankheiten des Immunsystems sowie viralen Erkrankungen verwendet werden. Die Verbindungen können in der jeweils geeigneten Formulierung enteral oder parenteral in Dosen von 1 bis 500 mg/kg, bevorzugt 1 bis 50 mg/kg verabreicht werden. Aufgrund der aufgefundenen guten bis hervorragenden Selektivität gegenüber PKC im Vergleich zu anderen Proteinkinasen besitzen die erfindungsgemäßen Verbindungen sehr viel weniger Nebenwirkungen beim Einsatz gegen die genannten Krankheiten.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citrat-Puffer, Ethanol, Komplexbildner (wie Äthylendiamintetraessigsäure und deren nicht-toxische Salze) sowie hochmolekulare Polymere (wie flüssiges Polyäthylenoxid) zur Viskositätsregulierung. Feste Trägerstoffe sind z.B. Stärke, Lactose, Mannit, Methylcellulose, Talkum, hochdisperse Kieselsäuren, höher molekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacksund/oder Süßstoffe enthalten.

Die folgenden Beispiele dienen der näheren Erläuterung der Erfindung.

### Beispiel 1

### 2-(1H-Indol-3-yl)-3-(1-methyl-1H-indol-3-yl)-maleinimid

0,3 g (0,87 mmol) 2-(lH-Indol-3-yl)-3-(1-methyl-lH-indol-3-yl)maleinsäureanhydrid und 15 g Ammoniumacetat werden 30 min. auf 140°C erhitzt. Nach dem Abkühlen wird die Reaktionsmischung mit viel Wasser verrührt, der rote Niederschlag wird abgesaugt, mit Wasser gut ausgewaschen und im Vakuum bei 13,3 Pa (0,1 Torr) und 120°C getrocknet. Man erhält 0,25 g (83%) rotes Produkt vom Schmelzpunkt 210°C (zers.). RF = 0,40 (Toluol:Ethanol = 10:2).

Das als Ausgangsmaterial verwendete 2-(1H-Indol-3-yl)-3-(1-methyl-lH-indol-3-yl)maleinsäureanhydrid wird wie folgt hergestellt: 0,5 g (1,4 mmol) 2-(1H-Indol-3-yl)-3-(1-methyl-1H-indol-3-yl)-N-methylmaleinimid und eine Lösung von 10 g Kaliumhydroxid in 100 ml Methanol/Wasser (1+1) werden 1 Stunde unter Rückfluß erhitzt. Nach dem Abkühlen wird die rote Reaktionsmischung mit halbkonz. Salzsäure angesäuert und mit Chloroform extrahiert. Nach dem Trocknen über Natriumsulfat wird das Extraktionsmittel am Rotavapor entfernt und der Rückstand mit wenig Toluol verrieben. Das rote Produkt wird abgesaugt und im Vakuum bei 13,3 Pa (0,1 Torr) und 70°C getrocknet. Man erhält 0,38 g (79%) rote Kristalle vom Schmelzpunkt 234°C (zers.). RF = 0,60 (Toluol:Ethanol = 10:1).

Das als Ausgangsmaterial verwendete 2-(1H-Indol-3-yl)-3-(1-methyl-1H-indol-3-yl)-N-methylmaleinimid wird wie folgt hergestellt:

Eine Lösung von 9,1 mmol Ethylmagnesiumbromid in 10 ml Tetrahydrofuran wird mit einer Lösung von 1,1 g (9,1 mmol) Indol in 10 ml Tetrahydrofuran versetzt. Man rührt 1 Stunde bei Raumtemperatur, versetzt mit einer Lösung von 1,2 g (3,7 mmol) 2-Brom-3-(1-methyl-lH-indol-3-yl)-N-methylmaleinimid in 10 ml Tetrahydrofuran und erhitzt 4 Stunden unter Rückfluß. Man läßt abkühlen, zersetzt die Reaktionsmischung mit einer gesättigten Lösung von Ammoniumchlorid in Wasser und extrahiert mit Essigester. Nach dem Trocknen über Natriumsulfat wird der Essigester am Rotavapor entfernt und der Rückstand an Kieselgel (Merck Art. 7734) flash chromatographiert, als Elutionsmittel wird eine Mischung aus Toluol und Ethanol (10+0,5) verwendet. Zuerst werden nicht umgesetzte Ausgangsprodukte eluiert, dann das gewünschte Produkt. Die produkthaltigen Fraktionen werden vereinigt, das Elutionsmittel wird abgezogen und der Rückstand mit wenig Toluol/Petrolether verrieben. Man erhält nach dem Absaugen und Trocknen im Vakuum 13,3 Pa (0,1 Torr)/ 70°C) 0,6 g rote Kristalle (45%) vom Schmelzpunkt 205°C. RF = 0,32 (Toluol+Ethanol = 10+2).

Das als Ausgangsmaterial verwendete 2-Brom-3-(1-methyl 1H-indol-3-yl)-N-methylmaleinimid wird wie folgt hergestellt:

1,4 g (5 mmol) 2-Brom-3-(1H-indol-3-yl)maleinimid werden in 20 ml Dimethylformamid gelöst und unter Stickstoff vorsichtig mit 0,3 g Natriumhydrid (80%ige Suspension in Paraffinöl) versetzt. Man rührt 1 Stunde bei Raumtemperatur weiter, versetzt mit einer Lösung von 1,6 g (11,2mmol) Methyljodid in 5 ml Dimethylformamid und erwärmt 6 Stunden auf 60°C. Nach dem Abkühlen wird das Lösungsmittel im Wasserstrahlvakuum abdestilliert und der Rückstand mit Wasser verrieben. Der orangefarbene Niederschlag wird abgesaugt und aus wenig Methanol kristallisiert.

Man erhält 1,3 g (81%) orangefarbene Kristalle vom Schmelzpunkt 167°C. RF = 0,48 (Hexan:Ethylacetat = 1:1).

Das als Ausgangsmaterial verwendete 2-Brom-3-(1H-indol-3-yl)-maleinimid wird wie folgt erhalten:

Eine Lösung von 49,3 mmol Ethylmagnesiumbromid in 30 ml Tetrahydrofuran wird mit einer Lösung von 5,8 g (49,5 mmol) Indol in 50 ml Toluol versetzt. Man rührt 1 Stunde bei Raumtemperatur, tropft dann langsam eine Lösung von 2,5 g (9,8 mmol) Dibrommaleinimid in 20 ml Tetrahydrofuran / 50 ml Toluol hinzu und erhitzt 30 Stunden unter Rückfluß. Nach dem Abkühlen wird die Reaktionsmischung mit einer gesättigten Lösung von Ammoniumchlorid in Wasser zersetzt, die Produkte werden mit Essigester extrahiert und die organische Phase wird schließlich über Natriumsulfat getrocknet. Nach dem Einengen wird der rote Rückstand mit wenig Dichlormethan verrieben, abgesaugt und im Vakuum bei 13,3 Pa (0,1 Torr) und 70°C getrocknet. Man erhält 2,2 g (75%) orangefarbenes Produkt vom Schmelzpunkt 196°C. RF = 0,35 (Hexan:Ethylacetat = 1:1).

### Beispiel 2

### 2-(1H-Indol-3-yl)-3-[1-(3-dimethylaminopropyl)-1H-indol-3-yl]-maleinimid

0,20 g (0,44 mmol) 2-(1H-Indol-3-yl)-3-[1-(3-dimethylaminopropyl)-1H-indol-3-yl]maleinsäureanhydrid Hydrochlorid und 10 g Ammoniumacetat werden 30 min. auf 140°C erhitzt. Nach dem Abkühlen wird mit Wasser versetzt und mit Essigester extrahiert. Die organische Phase wird mit Natriumbicarbonatlösung und danach mit Wasser gut ausgewaschen und über Natriumsulfat getrocknet. Der Essigester wird abrotiert, der rote Rückstand mit wenig Wasser verrührt und das ausgefallene rote Produkt abgesaugt. Nach dem Trocknen im Vakuum bei 13,3 Pa (0,1 Torr)/ 140°C erhält man 0,165 g (91%) rotes Produkt vom Schmelzpunkt ca. 280°C (zers.). RF = 0,16 (Chloroform:Methanol = 10:2).

Das als Ausgangsmaterial verwendete 2-(1H-Indol-3-yl)-3-[1-(3-dimethylaminopropyl)-1H-indol-3-yl]maleinsäureanhydrid Hydrochlorid wird wie folgt hergestellt:

0,5 g (0,95 mmol) 2-(1-tert.-Butoxycarbonyl-1H-indol-3-yl)-3-[1-(3-dimethylaminopropyl)-1H-indol-3-yl]-N-methylmaleinimid und 50 ml 10%ige Kaliumhydroxidlösung (Methanol/Wasser 1+1) werden 30 min. unter Rückfluß erhitzt. Nach dem Abkühlen wird vorsichtig mit konz. Salzsäure angesäuert und mit Chloroform extrahiert.

Die organische Phase wird mit Wasser ausgewaschen und zur Trockene eingeengt. Der rote Rückstand wird mit wenig Methanol/Toluol verrieben, abgesaugt und bei 80°C getrocknet. Man erhält 0,34 g (79,5%) dunkelrotes Produkt vom Schmelzpunkt 232-234°C (zers.).

Das als Ausgangsmaterial verwendete 2-(1-tert.-Butoxycarbonyl-1H-indol-3-yl)-3-[1-(3-dimethylaminopropyl)-1H-indol-3-yl]-N-methylmaleinimid wird wie folgt hergestellt:

Eine Mischung aus 0,9 g (2,03 mmol) 2-(1-tert.-Butoxycarbonyl-1H-indol-3-yl)-3-(lH-indol-3-yl)-N-methylmaleinimid (Tetrahedron 44, 2887, 1988), 0,3 g Kaliumcarbonat und 0,3 g (2,4 mmol) 3-Dimethylaminopropylchlorid in 10 ml Aceton wird 12 Stunden bei Raumtemperatur und 8 Stunden unter Rückfluß gerührt. Nach dem Abkühlen wird die Reaktionsmischung in Wasser gegossen und mit Essigester extrahiert. Der rote Extrakt wird eingeengt und der Rückstand an Kieselgel (Merck Art. 7734) flash chromatographiert. Als Elutionsmittel wird eine Mischung aus Toluol/Ethanol 10+1,5 verwendet. Das gewünschte Produkt wird als rotes Öl erhalten und so in obige Reaktion eingesetzt. Ausbeute 0,8 g (74%). RF = 0,20 (Toluol:Ethanol = 1:2).

### Beispiel 3

### 2-(5-Hydroxy-1H-indol-3-yl)-3-(1H-indol-3-yl)maleinimid

0,15 g (0,34 mmol) 2-(5-Benzyloxy-1H-indol-3-yl)-3-(1H-indol-3-yl)maleinimid und 4,0 g (34 mmol) Pyridinium Hydrochlorid werden unter Stickstoff 1 Stunde auf 150°C erhitzt. Nach dem Abkühlen wird die Reaktionsmischung mit Eiswasser verdünnt und mit Essigester extrahiert. Die organische Phase wird mit Wasser ausgewaschen und zur Trockene eingeengt. Der feste Rückstand wird mit wenig Wasser verrührt, abgesaugt und im Vakuum bei 13,3 Pa (0,1 Torr), 80°C getrocknet. Man erhält 85 mg (73%) dunkelrotes Produkt vom Schmelzpunkt 200-205°C. RF = 0,29 (Toluol/Ethanol = 10:2).

### Beispiel 4

### 2-(5-Benzyloxy-1H-indol-3-yl)-3-(1H-indol-3-yl)maleinimid

0,7 g (1,6 mmol) 2-(5-Benzyloxy-1H-indol-3-yl)-3-(1H-indol-3-yl)maleinsäureanhydrid und 9,5 g Ammoniumacetat werden 30 min. auf 140°C erhitzt. Nach dem Abkühlen wird die Reaktionsmischung mit Wasser verrührt, der ausgefallene Niederschlag wird abgesaugt, gut mit Wasser ausgewaschen und im Vakuum bei 13,3 Pa (0,1 Torr) 80°C getrocknet. Man erhält 0,54 g (78%) orangefarbenes Produkt vom Schmelzpunkt 308-312°C. RF = 0,29
(Hexan:Ethylacetat = 1:1).

Das als Ausgangsmaterial verwendete 2-(5-Benzyloxy-1H-indol-3-yl)-3-(lH-indol-3-yl)maleinsäureanhydrid wird wie folgt hergestellt:

Eine Lösung von 1,0 g (1,83 mmol) 2-(5-Benzyloxy-1H-indol-3-yl)-3-(1-tert.-butoxycarbonyl-lH-indol-3-yl)-N-methylmaleinimid in 100 ml 10%iger Kaliumhydroxidlösung (Methanol/Wasser 1+1) wird 1 Stunde unter Rückfluß erhitzt. Nach dem Abkühlen wird die Reaktionsmischung vorsichtig mit halbkonz. Salzsäure angesäuert, wobei ein roter Niederschlag ausfällt. Der Niederschlag wird abgesaugt, gut mit Wasser ausgewaschen und an der Luft getrocknet. Man erhält 0,7 g (87%) orangefarbenes Produkt, Schmelzpunkt 236°C. RF = 0,43 (Hexan:Ethylacetat = 1:1).

Das als Ausgangsmaterial verwendete 2-(5-Benzyloxy-1H-indol-3-yl)-3-(1-tert.-butoxycarbonyl-1H-indol-3-yl)-N-methylmaleinimid wird wie folgt hergestellt:

Eine Lösung von 20 mmol Ethylmagnesiumbromid in 30 ml Tetrahydrofuran wird mit einer Lösung von 4,46 g (20 mmol) 5-Benzyloxyindol in 20 ml Tetrahydrofuran versetzt. Man hält die Reaktionsmischung 1 Stunde bei 50°C und tropft dann innerhalb 1 Stunde eine Lösung von 3,0 g (7,4 mmol) 2-Brom-3-[1-tert.-butoxycarbonyl-lH-indol-3-yl]-N-methylmaleinimid (Tetrahedron 44, 1988, 2887) in 40 ml Tetrahydrofuran hinzu. Man erhitzt 2 Stunden unter Rückfluß, läßt abkühlen und zersetzt die Reaktionsmischung mit einer gesättigten Lösung von Ammoniumchlorid in Wasser. Man extrahiert die Reaktionsprodukte mit Essigester, trocknet die organische Phase über Natriumsulfat und engt zur Trockene ein. Der Rückstand wird an Kieselgel (Merck Art. 7734) flash chromatographiert, als Elutionsmittel wird Dichlormethan verwendet. Nach Elution von nicht umgesetztem Ausgangsmaterial wird das gewünschte Produkt durch Einengen der entsprechenden Fraktionen und Verrühren des roten Rückstandes mit wenig Toluol erhalten. Man isoliert 1,3 g (32%) rote Kristalle vom Schmelzpunkt 123°C.

### Beispiel 5

### 2,3-Bis(5-methoxy-1H-indol-3-yl)maleinimid

Eine Lösung von 22 mmol Ethylmagnesiumbromid in 30 ml Tetrahydrofuran wird bei Raumtemperatur mit einer Lösung von 2,8 g (19 mmol) 5-Methoxyindol in 50 ml Toluol versetzt und 1 Stunde bei Raumtemperatur gerührt. Unter Stickstoff tropft man dann eine Lösung von 1,5 g (5,8 mmol) Dibrommaleinimid in 20 ml Tetrahydrofuran / 50 ml Toluol hinzu und erhitzt 20 Stunden unter Rückfluß. Nach dem Abkühlen wird die Reaktionsmischung mit einer gesättigten Lösung von Ammoniumchlorid in Wasser zersetzt, die Produkte werden mit Essigester extrahiert und die organische Phase über Natriumsulfat getrocknet. Nach dem Einengen wird der rote Rückstand mit wenig Methanol verrieben, das ausgefallene rote Produkt wird abgesaugt und im Vakuum bei 13,3 Pa (0,1 Torr)und 100°C getrocknet. Man erhält 0,9 g (36%) rote Kristalle, Schmelzpunkt 336°C unter Zersetzung. RF = 0,30 (Toluol:Ethanol = 10:2).

### Beispiel 6

### 2-(lH-Indol-3-yl)-3-(5,6-methylendioxy-1H-indol-3-yl)maleinimid

0,8 g (=2,1 mmol) 2-(1H-Indol-3-yl)-3-(5,6-methylendioxy-1H-indol-3-yl)-maleinsäureanhydrid werden zusammen mit 20 g Ammoniumacetat 1h auf 140°C (= Schmelze) erhitzt. Nach dem Abkühlen versetzt man mit 150 ml Wasser und saugt den rotbraunen Niederschlag ab. Zur Reinigung wird an Kieselgel mit CH₂Cl₂ : EtOAc = 3:1 als Laufmittel chromatographiert.

Man erhält 0,40 g (= 45% d. Th.) 2-(lH-Indol-3-yl)-3-(5,6-methylendioxy-1H-indol-3-yl)maleinimid als rote Kristalle vom Schmp. 150°C (Zers.) und RF = 0.4 (CH₂Cl₂:EtOAc = 3:1).

Das als Ausgangsmaterial verwendete 2-(1H-Indol-3-yl)-3-(5,6-methylendioxy-1H-indol-3-yl)maleinsäureanhydrid wird wie folgt hergestellt:

4,0 g (= 10,4 mmol) 2-(1H-Indol-3-yl)-3-(5,6-methylendioxy-1H-indol-3-yl)-N-methylmaleinimid werden zusammen mit einer Lösung von 160 g KOH in 1600 ml Wasser 30 min am Rückfluß erhitzt. Die zuerst rote Suspension geht dabei in eine braune Lösung über. Man läßt abkühlen, säuert mit konz. HCl an und saugt den roten Niederschlag ab.

Man erhält 3,40 g (= 88% d. Th.) 2-(1H-Indol-3-yl)-3-(5,6-methylendioxy-1H-indol-3-yl)-maleinsäureanhydrid, hellrote Kristalle (aus Methanol) vom Schmp. ≈ 200°C (Zers.) und RF = 0.70 (CH₂Cl₂ : EtOAc = 3:1).

Das als Ausgangsmaterial verwendete 2-(lH-Indol-3-yl)-3-(5,6-methylendioxy-1H-indol-3-yl)-N-methylmaleinimid wird wie folgt hergestellt:

8,06 g (= 50 mmol) 5,6-Methylendioxyindol werden in 400 ml Toluol gelöst und bei 60°C mit 25 ml einer 2 M Lösung von EtMgBr in THF (Aldrich) versetzt. Nach 1 h bei 60°C tropft man eine Lösung von 8,1 g (= 20 mmol) 2-Brom-3-(1-tert.-butyloxycarbonyl-1H-indol-3-yl)-N-methylmaleinimid in 100 ml Toluol hinzu und erhitzt 3h am Rückfluß. Nach dem Abkühlen auf Raumtemperatur hydrolysiert man durch Zugabe von 100 ml ges. Ammoniumchlorid-Lösung. Man trennt die org. Phase ab, schüttelt die wäßrige Phase mit Essigester aus, löst die Rückstände in Essigester, trocknet die vereinigten organischen Phasen, engt ein und chromatographiert den Rückstand an 600 g Kieselgel mit CH₂Cl₂ : EtOAc = 3:1 als Laufmittel.

Man isoliert 3,75 g 5,6-Methylendioxyindol vom Schmp. 104-106°C, 0,55 g 2-Brom-3-(1H-indol-3-yl)-N-methylmaleinimid und 7,27 g (94 % d.Th.) von 2-(1H-indol-3-yl)-3-(5,6-methylendioxy-1H-indol-3-yl)-N-methylmaleinimid als rote Kristalle vom Schmp. 140°C (Zers.) und RF = 0,6 (CH₂Cl₂ : EtOAc = 3:1).

### Beispiel 7

### 2-(1H-Indol-3-yl)-3-(5,6-dimethoxy-1H-indol-3-yl)maleinimid

3,0 g (= 7,72 mmol) 2-(1H-indol-3-yl)-3-(5,6-Dimethoxy-1H-indol-3-yl)maleinsäureanhydrid werden zusammen mit 75 g Ammoniumacetat 1 h auf 140°C erhitzt. Nach dem Abkühlen versetzt man mit 1,5 1 Wasser und saugt den dunkelroten Niederschlag ab. Man erhält 2,61 g Rohausbeute. Zur Reinigung werden davon 1.75 g an Kieselgel mit CH₂Cl₂ : EtOAc = 8:1 als Laufmittel chromatographiert.

Man erhält 1,20 g 2-(1H-indol-3-yl)-3-(5,6-Dimethoxy-1H-indol-3-yl)maleinimid 1/2 EtOAc als dunkelrote Kristalle vom Schmp. 120°C (Zers.) und RF = 0.32 (CH₂Cl₂ : EtOAc = 3:1). Ausbeute: 54% d. Th.

Das als Ausgangsmaterial verwendete 2-(1H-Indol-3-yl)-3-(5,6-dimethoxy-1H-indol-3-yl)maleinsäureanhydrid wird wie folgt hergestellt:

10 g (= 25 mmol) 2-(1H-indol-3-yl)-3-(5,6-dimethoxy 1H-indol-3-yl)-N-methylmaleinimid werden in 4000 ml 10%iger KOH 30 min am Rückfluß erhitzt. Die zuerst rote Suspension geht dabei in eine braune Lösung über. Nach dem Abkühlen säuert man mit konz. HCl an und saugt dem roten Niederschlag ab. Man erhitzt den Niederschlag in Methanol und filtriert.

Man erhält 8,48 g 2-(1H-Indol-3-yl)-3-(5,6-dimethoxy 1H-indol-3-yl)maleinsäureanhydrid (= 87,6 % d.Th.) als dunkelrote Kristalle vom Schmp. ≈ 320°C (Zers.) und RF = 0.50 (CH₂Cl₂ : EtOAc = 3:1).

Das als Ausgangsmaterial verwendete 2-(1H-Indol-3-yl)-3-(5,6-dimethoxy-1H-indol-3-yl)-N-methylmaleinimid wird wie folgt hergestellt:

22,15 g (= 0,125 m) 5,6-Dimethoxyindol werden in 1000 ml Toluol gelöst und bei 40°C mit 62,5 ml einer 2 M Lösung von EtMgBr in THF (Aldrich) versetzt. Nach 1 h bei 60°C tropft man eine Lösung von 20,26 g (= 0,05 m) 2-Brom-3-(1-tert.butyloxycarbonyl-1H-indol-3-yl)-N-methylmaleinimid in 250 ml Toluol hinzu und erhitzt anschließend 3 h am Rückfluß. Nach dem Abkühlen hydrolysiert man durch Zugabe von ges. Ammoniumchlorid-Lösung. Der gebildete rote Niederschlag wird abfiltriert und an Kieselgel mit CH₂Cl₂:EtOAc = 10:1 als Laufmittel chromatographiert.

Man isoliert 6,51 g von 2-(1H-Indol-3-yl)-3-(5,6-dimethoxy-1H-indol-3-yl)-N-methylmaleinimid (= 32% d.Th.) als rote Kristalle vom Schmp. 258°C und RF = 0.44 (CH₂Cl₂ : EtOAc = 3:1) und 7,16 g (≈ 35 % d.Th.) rote Kristalle der gleichen, aber leicht verunreinigten Verbindung mit dem Schmp. 250-254°C.

### Beispiel 8

### 2,3-Bis-(5,6-methylendioxy-1H-indol-3-yl)maleinimid

6,44 g (= 40 mmol) 5,6-Methylendioxyindol werden in 300 ml abs. Toluol gelöst und bei 60°C tropfenweise mit 20 ml einer 2 M Lösung von EtMgBr in THF (Aldrich) versetzt. Nach beendeter Zugabe tropft man eine Lösung von 1,7 g (= 6,67 mmol) Dibrommaleinimid in 50 ml Toluol (unter Zusatz der zur Lösung benötigten Minimalmenge an abs. THF) hinzu und erhitzt 18 h am Rückfluß. Nach dem Abkühlen versetzt man mit ges.
Ammoniumchlorid-Lösung, trennt die organische Phase ab, extrahiert die wäßr. Phase 3 mal mit Toluol und 1 mal mit Essigester. Die vereinigten org. Phasen werden getrocknet, einrotiert und an Kieselgel chromatographiert (Laufmittel: CH₂Cl₂: EtOAc = 30:1).

Man isoliert 1,27 g (= 56 % d.Th.) 2-Brom-3-(5,6-methylendioxy-lH-indol-3-yl)maleinimid vom Schmp. ≈ 185°C (Zers.) und RF = 0.50 (CH₂Cl₂ : EtOAc = 3:1) und 0,63 g (= 22,5% d.TH) 2,3-Bis(5,6-methylendioxy-1H-indol-3-yl)maleinimid als rote Kristalle vom Schmp. ≈ 270°C (Zers.) (aus Propanol) und RF = 0.36 (CH₂C₁₂ : EtOAc = 3:1).

### Beispiel 9

2,3-Bis-(1H-indol-3-yl)maleinimid wurde nach Literatur (Tetrahedron 44, 1988, 2887) erhalten.

### Beispiel 10

2-(6-Hydroxy-1H-indol-3-yl)-3-(1H-indol-3-yl)maleinimid wurde nach Literatur (Tetrahedron 44, 1988, 2887) erhalten.

In analoger Weise zu den Beispielen 1 bis 10 und den angegebenen Verfahren erhält man:
11.) 2-(5-Benzyloxy-1H-indol-3-yl)-3-[1-(3-dimethylaminopropyl)-1H-indol-3-yl]maleinimid, 188-189°C;
12.) 2-[5-Benzyloxy-1-(3-dimethylaminopropyl)-1H-indol-3-yl]-3-(lH-indol-3-yl)maleinimid, 240-241°C;
13.) 2-(5-Methoxy-1H-indol-3-yl)-3-[5-methoxy-1-(3-dimethylaminopropyl)-1H-indol-3-yl]maleinimid, 250-251°C;
14.) (±)-2-(1H-Indol-3-yl)-3-[1-(3-dimethylamino-2-methoxypropyl)-1H-indol-3-yl]maleinimid, 218°C, Zers.;
15.) 2-(1H-Indol-3-yl)-3-[5-(3-dimethylaminopropyloxy)-1H-indol-3-yl]maleinimid, 235°C;
16.) 2,3-Bis-(5-fluor-1H-indol-3-yl)maleinimid, 320°C, Zers.;
17.) 2-(5-Fluor-1H-indol-3-yl)-3-[5-fluor-1-(3-dimethylaminopropyl)-1H-indol-3-yl]maleinimid, 283-285°C;
18.) 2,3-Bis-(5-benzyloxy-1H-indol-3-yl)maleinimid, 130°C, Zers.;
19.) 2-(5-Benzyloxy-1H-indol-3-yl)-3-[5-benzyloxy-1-(3-dimethylaminopropyl-1H-indol-3-yl]maleinimid, 277°C, Zers.;
20.) 2-(5-Benzyloxy-1-methyl-1H-indol-3-yl)-3-(1-methyl-1H-indol-3-yl)maleinimid;
21.) 2-[5-Benzyloxy-1-(3-dimethylaminopropyl)-1H-indol-3-yl]-3-(1-methyl-1H-indol-3-yl)maleinimid, 181-182°C;
22.) 2,3-Bis-(5-chlor-1H-indol-3-yl)maleinimid, 279-281°C, Zers.;
23.) 2-(5-Chlor-1H-indol-3-yl)-3-[5-chlor-1-(3-dimethylaminopropyl)-1H-indol-3-yl]maleinimid, 255-257°C, Zers.;
24.) 2,3-Bis-(5-methyl-1H-indol-3-yl)maleinimid, 180°C, Zers.;
25.) 2-(5-Methyl-1H-indol-3-yl)-3-[5-methyl-1-(3-dimethylaminopropyl)-1H-indol-3-yl]maleinimid, 215°C;
26.) 2,3-Bis-(5-brom-1H-indol-3-yl)maleinimid, 351°C;
27.) 2-(5-Brom-1H-indol-3-yl)-3-[5-brom-1-(3-dimethylaminopropyl)-1H-indol-3-yl]maleinimid, 257-258°C;
28.) 2-(1H-Indol-3-yl)-3-[1-(2-dimethylaminoethyl)-1H-indol-3-yl]-maleinimid, hellrote Kr., 246-248°C, zers., hergestellt nach Verfahren C.
29.) 2-(1H-Indol-3-yl)-3-[1-(3-(1-piperidino)propyl)-1H-indol-3-yl]-maleinimid, hellorange Kr., 204°C hergestellt nach Verfahren C.
30.) 2-(1H-Indol-3-yl)-3-[1-(3-(4-morpholino)propyl)-1H-indol-3-yl]-maleinimid, hellorange Kr., 285-287°C, zers., hergestellt nach Verfahren C.
31.) 2-[1-(3-Diethylaminopropyl)-1H-indol-3-yl]-3-(1H-indol-3-yl)-maleinimid, hellorange Kr., 241-242°C, zers., hergestellt nach Verfahren C.
32.) (±)-2-[1-(3-Diethylamino-2-hydroxypropyl)-1H-indol-3-yl]-3-(1H-indol-3-yl)-maleinimid, dunkelrote Kr., 158°C, hergestellt nach Verfahren C unter Verwendung von 1,1-Diethyl-3-hydroxyazetidiniumchlorid als Alkylierungsmittel.
33.) 2-(5-Methoxy-1H-indol-3-yl)-3-[1-(3-dimethylaminopropyl)-1H-indol-3-yl]-maleinimid, hellorange Kr., 224-225°C, hergestellt nach Verfahren C unter Verwendung der SEM-Schutzgruppe.
34.) 2-(1H-Indol-3-yl)-3-[5-methoxy-1-(3-dimethylaminopropyl)-1H-indol-3-yl]-maleinimid, hellrote Kr., 234-236°C, hergestellt nach Verfahren B unter Verwendung der SEM-Schutzgruppe.
35.) (±)-2-[1-(2,3-Dihydroxypropyl)-1H-indol-3-yl]-3-(1H-indol-3-yl)-maleinimid, dunkelrote Kr., 135°C, hergestellt nach Verfahren B unter Verwendung der BOC-Schutzgruppe und Epibromhydrin als Alkylierungsmittel.
36.) 2-(5-Methoxy-1H-indol-3-yl)-3-[1-(3-(4-morpholino)propyl)-1H-indol-3-yl]-maleinimid und
37.) 2-(1H-Indol-3-yl)-3-[5-methoxy-1-(3-(4-morpholino)-propyl)-1H-indol-3-yl]-maleinimid, als Regioisomerenmischung im Verhältnis 1:1, orangefarb. Kr., 132-135°C, hergestellt nach Verfahren B unter Verwendung der BOC-Schutzgruppe.
38.) (±)-2-[1-(3-Ethoxy-2-hydroxypropyl)-1H-indol-3-yl]-3-(1H-indol-3-yl)-maleinimid, dunkelrote Kr., 130-134°C erhalten nach Verfahren B unter Verwendung der BOC-Schutzgruppe und Epibromhydrin als Alkylierungsmittel.
39.) (±)-2-[1-(2-Hydroxy-3-(1-piperidino)-propyl)-1H-indol-3-yl]-3-(1H-indol-3-yl)-maleinimid, dunkelrote Kr., 142°C, zers., hergestellt nach Verfahren B unter Verwendung der BOC-Schutzgruppe und Epibromhydrin als Alkylierungsmittel.
40.) (±)-2-[1-(3-Diethylamino-2-hydroxypropyl)-5-methoxy-1H-indol-3-yl]-3-(1H-indol-3-yl)-maleinimid und
41.) (±)-2-[1-(3-Diethylamino-2-hydroxypropyl)-1H-indol-3-yl]-3-(5-methoxy-1H-indol-3-yl)-maleinimid, als Regioisomerenmischung im Verhältnis 4:1, dunkelrote Kr., ab 150°C zers., hergestellt nach Verfahren B unter Verwendung der BOC-Schutzgruppe und 1,1-Diethyl-3-hydroxyazetidinium-chlorid als Alkylierungsmittel.
42.) 2-(1H-Indol-3-yl)-3-[5-methoxy-1-(3-(1-piperidino)propyl)-1H-indol-3-yl]-maleinimid, 257-258°C und zusammen mit
43.) 2-(5-Methoxy-1H-indol-3-yl)-3-[1-(3-(1-piperidino)propyl)-1H-indol-3-yl]-maleinimid, als Regioisomerenmischung im Verhältnis 1:1, orangefarb. Kr., 150°C, hergestellt nach Verfahren B unter Verwendung der BOC-Schutzgruppe.
44.) 2-[1-(3-Diethylaminopropyl)-5-methoxy-1H-indol-3-yl]-3-(5,6-dimethoxy-1-methyl-1H-indol-3-yl)-maleinimid, hellrote Kr., 165-166°C, hergestellt nach Verfahren A.
45.) 2-[1-(3-Diethylaminopropyl)-5-methoxy-1H-indol-3-yl]-3-(1H-indol-3-yl)-maleinimid und
46.) 2-[1-(3-Diethylaminopropyl)-1H-indol-3-yl)-3-(5-methoxy-1H-indol-3-yl)-maleinimid, als Regioisomerenmischung im Verhältnis 3:2, hellrote Kr., 206-208°C, zers., hergestellt nach Verfahren B unter Verwendung der BOC-Schutzgruppe.
47.) 2-[1-(3-Diethylaminopropyl)-1H-indol-3-yl]-3-(5-methoxy-1H-indol-3-yl)-maleinimid und
48.) 2-[1-(3-Diethylaminopropyl)-5-methoxy-1H-indol-3-yl]-3-(1H-indol-3-yl)-maleinimid, als Regioisomerenmischung im Verhältnis 3:1, hellrote Kr., 185-190°C, hergestellt nach Verfahren B.
49.) 2-[1-(3-Diethylaminopropyl)-5-methoxy-1H-indol-3-yl]-3-(6-methoxy-1H-indol-3-yl)-maleinimid und
50.) 2-[1-(3-Diethylaminopropyl)-6-methoxy-1H-indol-3-yl]-3-(5-methoxy-1H-indol-3-yl)-maleinimid, als Regioisomerenmischung im Verhältnis 5:1, rote Kr., 176-182°C, hergestellt nach Verfahren B.
51.) 2-[5-Fluor-1-(3-dimethylaminopropyl)-1H-indol-3-yl]-3-(1H-indol-3-yl)-maleinimid und
52.) 2-(5-Fluor-1H-indol-3-yl)-3-[1-(3-dimethylaminoplopyl)-1H-indol-3-yl]-maleinimid, als Regioisomerenmischung im Verhältnis 3:1, 230-235°C.

In entsprechender Weise werden die folgenden Verbindungen je nach Wahl der Substituenten nach einem der genannten Verfahren hergestellt:
2-(5-Brom-1H-indol-3-yl)-3-(1H-indol-3-yl)-maleinimid;
2-(5-Fluor-1H-indol-3-yl)-3-(1H-indol-3-yl)-maleinimid;
2-[1-(3-Diethylaminopropyl)-5-fluor-1H-indol-3-yl]-3-(1H-indol-3-yl) -maleinimid;
(±)-2-[1-(2,3-Dihydroxypropyl)-S-methoxy-1H-indol-3-yl]-3-(1H-indol-3-yl)-maleinimid; und
(±)-2-[1-(3-Ethoxy-2-hydroxypropyl)-5-methoxy-1H-indol-3-yl]-3-(1H-indol-3-yl)-maleinimid;
sowie die aus den Racematen isolierten Enantiomere der Verbindungen:
(+)-2-[1-(3-Diethylamino-2-hydroxypropyl)-1H-indol-3-yl]-3-(1H-indol-3-yl)-maleinimid;
(-)-2-[1-(3-Diethylamino-2-hydroxypropyl)-1H-indol-3-yl]-3-(1H-indol-3-yl)-maleinimid;
(+)-2-[1-(2,3-Dihydroxypropyl)-1H-indol-3-yl]-3-(1H-indol-3-yl)-maleinimid;
(-)-2-[1-(2,3-Dihydroxypropyl)-1H-indol-3-yl]-3-(1H-indol-3-yl)-maleinimide;
(+)-2-[1-(3-Ethoxy-2-hydroxypropyl)-1H-indol-3-yl]-3-(1H--indol-3-yl)-maleinimid;
(-)-2-[1-(3-Ethoxy-2-hydroxypropyl)-1H-indol-3-yl]-3-(1H-indol-3-yl) -maleinimid;
(+)-2-[1-(2-Hydroxy-3-(1-piperidino)-propyl)-1H-indol-3yl]-3-(1H-indol-3-yl)-maleinimid;
(-)-2-[1-(2-Hydroxy-3-(1-piperidino)-propyl)-1H-indol-3yl]-3-(1H-indol-3-yl)-maleinimid;
(+)-2-[1-(3-Diethylamino-2-hydroxypropyl)-5-methoxy-1H-indol-3-yl]-3-(1H-indol-3-yl)-maleinimid;
(-)-2-[1-(3-Diethylamino-2-hydroxypropyl)-5-methoxy-1H--indol-3-yl]-3-(1H-indol-3-yl)-maleinimid;
(+)-2-[1-(3-Diethylamino-2-hydroxypropyl)-1H-indol-3-yl]-3-(5-methoxy-1H-indol-3-yl)-maleinimid;
(-)-2-[1-(3-Diethylamino-2-hydroxypropyl)-1H-indol-3-yl]-3-(5-methoxy-1H-indol-3-yl)-maleinimid;
(+)-2-[1-(2,3-Dihydroxypropyl)-5-methoxy-1H-indol-3-yl]-3-(1H-indol-3-yl)-maleinimid;
(-)-2-[1-(2,3-Dihydroxypropyl)-5-methoxy-1H-indol-3-yl]-3-(1H-indol-3-yl)-maleinimid;
(+)-2-[1-(3-Ethoxy-2-hydroxypropyl)-5-methoxy-1H-indol-3-yl]-3-(1H-indol-3-yl)-maleinimid; und
(-)-2-[1-(3-Ethoxy-2-hydroxypropyl)-5-methoxy-1H-indol-3-yl]-3-(1H-indol-3-yl)-maleinimid.

## Patentansprüche

1. Bis-(1H-indol-3-yl)maleinimid-Derivate der allgemeinen Formel I, 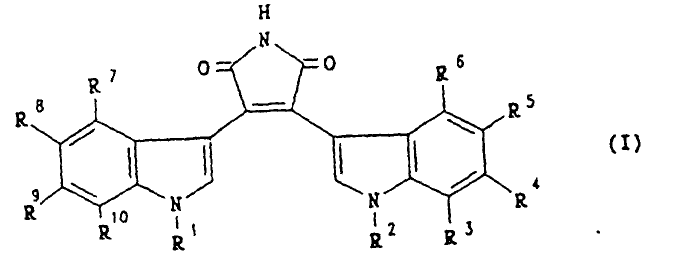 nämlich:
2-(1H-Indol-3-yl)-3-(1-methyl-1H-indol-3-yl)-maleinimid
2-(1H-Indol-3-yl)-3-[1-(3-dimethylaminopropyl)-1H-indol-3-yl]-maleinimid
2-(5-Hydroxy-1H-indol-3-yl)-3-(1H-indol-3-yl)maleinimid
2-(5-Benzyloxy-1H-indol-3-yl)-3-(1H-indol-3-yl)maleinimid
2,3-Bis(5-methoxy-1H-indol-3-yl)maleinimid
2-(1H-Indol-3-yl)-3-(5,6-methylendioxy-1H-indol-3-yl)maleinimid
2-(1H-Indol-3-yl)-3-(5,6-dimethoxy-1H-indol-3-yl)maleinimid
2,3-Bis-(5,6-methylendioxy-1H-indol-3-yl)maleinimid
2-(1H-Indol-3-yl)-3-(5-methoxy-1H-indol-3-yl)maleinimid
2-(5-Benzyloxy-1H-indol-3-yl)-3-[1-(3-dimethylaminopropyl)-1H-indol-3-yl]maleinimid
2-[5-Benzyloxy-1-(3-dimethylaminopropyl)-1H-indol-3-yl]-3-(1H-indol-3-yl)maleinimid
2-(5-Methoxy-1H-indol-3-yl)-3-[5-methoxy-1-(3-dimethylaminopropyl)-1H-indol-3-yl]maleinimid
(±)-2-(1H-Indol-3-yl)-3-[1-(3-dimethylamino-2-methoxypropyl)-1H-indol-3-yl]maleinimid
2-(1H-Indol-3-yl)-3-[5-(3-dimethylaminopropyloxy)-1H-indol-3-yl]maleinimid
2,3-Bis-(5-fluor-1H-indol-3-yl)maleinimid
2-(5-Fluor-1H-indol-3-yl)-3-[5-fluor-1-(3-dimethylaminopropyl)-1H-indol-3-yl]maleinimid
2,3-Bis-(5-benzyloxy-1H-indol-3-yl)maleinimid
2-(5-Benzyloxy-1H-indol-3-yl)-3-[5-benzyloxy-1-(3-dimethylaminopropyl-1H-indol-3-yl]maleinimid
2-[5-Benzyloxy-1-(3-dimethylaminopropyl)-1H-indol-3-yl]-3-(1-methyl-1H-indol-3-yl)maleinimid
2,3-Bis-(5-chlor-1H-indol-3-yl)maleinimid
2-(5-Chlor-1H-indol-3-yl)-3-[5-chlor-1-(3-dimethylaminopropyl)-1H-indol-3-yl]maleinimid
2,3-Bis-(5-methyl-1H-indol-3-yl)maleinimid
2-(5-Methyl-1H-indol-3-yl)-3-[5-methyl-1-(3-dimethylaminopropyl)-1H-indol-3-yl]maleinimid
2,3-Bis-(5-brom-1H-indol-3-yl)maleinimid
2-(5-Brom-1H-indol-3-yl)-3-[5-brom-1-(3-dimethylaminopropyl)-1H-indol-3-yl]maleinimid
2-(1H-Indol-3-yl)-3-[1-(2-dimethylaminoethyl)-1H-indol-3-yl]-maleinimid;
2-(1H-Indol-3-yl)-3-[1-(3-(1-piperidino)propyl)-1H-indol-3-yl]-maleinimid;
2-(1H-Indol-3-yl)-3-[1-(3-(4-morpholino)propyl)-1H-indol-3-yl]-maleinimid;
2-[1-(3-Diethylaminopropyl)-1H-indol-3-yl]-3-(1H-indol-3-yl)-maleinimid;
(±)-2-[1-(3-Diethylamino-2-hydroxypropyl) -1H-indol-3-yl]-3-(1H-indol-3-yl)-maleinimid;
(+)-2-[1-(3-Diethylamino-2-hydroxypropyl)-1H-indol-3-yl]-3-(1H-indol-3-yl)-maleinimid;
(-)-2-[1-(3-Diethylamino-2-hydroxypropyl)-1H-indol-3-yl]-3-(1H-indol-3-yl)-maleinimid;
2-(5-Methoxy-1H-indol-3-yl)-3-[1-(3-dimethylaminopropyl)-1H-indol-3-yl]-maleinimid;
2-(1H-Indol-3-yl)-3-[5-methoxy-1-(3-dimethylaminopropyl)-1H-indol-3-yl]-maleinimid;
(±)-2-[1-(2,3-Dihydroxypropyl)-1H-indol-3-yl]-3-(1H-indol-3-yl)-maleinimid;
(+)-2-[1-(2,3-Dihydroxypropyl) -1H-indol-3-yl]-3-(1H-indol-3-yl)-maleinimid;
(-)-2-[1-(2,3-Dihydroxypropyl)-1H-indol-3-yl]-3-(1H-indol-3-yl) -maleinimid;
2-(5-Methoxy-1H-indol-3-yl)-3-[1-(3-(4-morpholino)propyl)-1H-indol-3-yl]-maleinimid;
2-(1H-Indol-3-yl)-3-[5-methoxy-1-(3-(4-morpholino)propyl)-1H-indol-3-yl]-maleinimid;
(±)-2-[1-(3-Ethoxy-2-hydroxypropyl)-1H-indol-3-yl]-3-(1H-indol-3-yl)-maleinimid;
(+)-2-[1-(3-Ethoxy-2-hydroxypropyl)-1H-indol-3-yl]-3-(1H-indol-3-yl)-maleinimid;
(-)-2-[1-(3-Ethoxy-2-hydroxypropyl)-1H-indol-3-yl]-3-(1H-indol-3-yl)-maleinimid;
(±)-2-[1-(2-Hydroxy-3-(1-piperidino)-propyl)-1H-indol-3-yl]-3-(1H-indol-3-yl)-maleinimid;
(+)-2-[1-(2-Hydroxy-3-(1-piperidino)-propyl)-1H-indol-3-yl]-3-(1H-indol-3-yl)-maleinimid;
(-)-2-[1-(2-Hydroxy-3-(1-piperidino)-propyl)-1H-indol-3-yl]-3-(1H-indol-3-yl)-maleinimid;
(±)-2-[1-(3-Diethylamino-2-hydroxypropyl)-5-methoxy-1H-indol-3-yl]-3-(1H-indol-3-yl)-maleinimid;
(+)-2-[1-(3-Diethylamino-2-hydroxypropyl)-5-methoxy-1H-indol-3-yl]-3-(1H-indol-3-yl)-maleinimid;
(-)-2-[1-(3-Diethylamino-2-hydroxypropyl)-5-methoxy-1H-indol-3-yl]-3-(1H-indol-3-yl)-maleinimid;
(±)-2-[1-(3-Diethylamino-2-hydroxypropyl)-1H-indol-3-yl]-3-(5-methoxy-1H-indol-3-yl)-maleinimid;
(+)-2-[1-(3-Diethylamino-2-hydroxypropyl)-1H-indol-3-yl]-3-(5-methoxy-1H-indol-3-yl)-maleinimid;
(-)-2-[1-(3-Diethylamino-2-hydroxypropyl)-1H-indol-3-yl]-3-(5-methoxy-1H-indol-3-yl)-maleinimid;
2-(1H-Indol-3-yl)-3-[5-methoxy-1-(3-(1-piperidino)propyl)-1H-indol-3-yl]-maleinimid;
2-(5-Methoxy-1H-indol-3-yl)-3-[1-(3-(1-piperidino)propyl)-1H-indol-3-yl]-maleinimid;
2-[1-(3-Diethylaminopropyl)-5-methoxy-1H-indol-3-yl]-3-(5,6-dimethoxy-1-methyl-1H-indol-3-yl)-maleinimid;
2-[1-(3-Diethylaminopropyl)-5-methoxy-1H-indol-3-yl]-3-(1H-indol-3-yl) -maleinimid;
2-[1-(3-Diethylaminopropyl)-1H-indol-3-yl)-3-(5-methoxy-1H-indol-3-yl) -maleinimid;
2-[1-(3-Diethylaminopropyl)-5-methoxy-1H-indol-3-yl]-3-(6-methoxy-1H-indol-3-yl)-maleinimid;
2-[5-Fluor-1-(3-dimethylaminopropyl)-1H-indol-3-yl]-3-(1H-indol-3-yl)-maleinimid;
2-(5-Fluor-1H-indol-3-yl)-3-[1-(3-dimethylaminoplopyl)-1H-indol-3-yl]-maleinimid.
2-[1-(3-Diethylaminopropyl)-6-methoxy-1H-indol-3-yl]-3-(5-methoxy-1H-indol-3-yl)-maleinimid;
2-(5-Bromo-1H-indol-3-yl)-3-(1H-indol-3-yl)-maleimid;
2-(5-Fluoro-1H-indol-3-yl)-3-(1H-indol-3-yl)-maleimid;
2-[1-(3-Diethylaminopropyl)-5-fluoro-1H-indol-3-yl]-3-(1H-indol-3-yl)-maleimid
(±)-2-[1-(2,3-Dihydroxypropyl)-5-methoxy-1H-indol-3-yl]-3-(1H-indol-3-yl)-maleimid
und
(±)-2-[1-(3-Ethoxy-2-hydroxypropyl)-5-methoxy-1H-indol-3-yl]-3-(1H-indol-3-yl)-maleimid,

2. Verfahren zur Herstellung von Verbindungen gemäß Ansprüch 1 **dadurch gekennzeichnet, daß** man entweder
A) ein Dibrommaleinimid der allgemeinen Formel II, 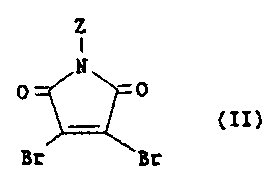 in welcher Z eine geeignete abspaltbare Schutzgruppe ist, mit einem Indol-Grignardreagenz der allgemeinen Formel A, 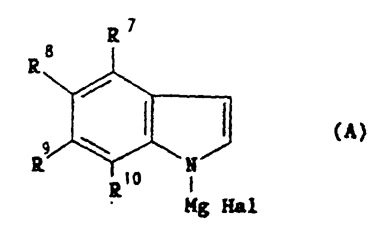 in welcher R⁷, R⁸, R⁹ und R¹⁰ die in den Einzelverbindungen genannte Bedeutung haben, nicht jedoch Hydroxy oder Acyloxy sind, nach an sich bekannten Methoden umsetzt, und das erhaltene Produkt der allgemeinen Formel III, 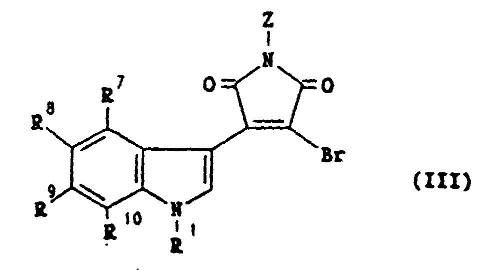 bei der R¹ Wasserstoff bedeutet, gegebenenfalls am Indolstickstoff mit einem Alkylierungsmittel R¹-X, wobei R¹ mit Ausnahme von Wasserstoff die in den Einzelverbindungen genannten Bedeutungen hat, nicht jedoch funktionelle Gruppen enthält, die die nachfolgenden Umsetzungen stören würden, und X für eine leicht austretende Gruppe wie Chlor oder Brom steht, in an sich bekannter Weise alkyliert, wobei ein Produkt der allgemeinen Formel III erhalten wird; bei dem R¹ verschieden von Wasserstoff ist, und anschließend Produkt III mit einem Indolgrignardreagenz der allgemeinen Formel (B), 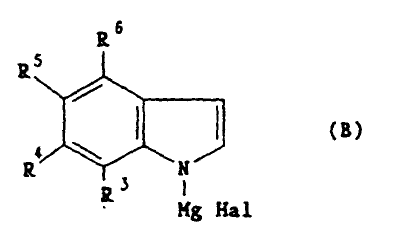 in welcher R³, R⁴, R⁵ und R⁶ die in den Einzelverbindungen genannte Bedeutung haben, nicht jedoch Hydroxy oder Acyloxy sind, umsetzt und gegebenenfalls anschließend mit einem Alkylierungsmittel der allgemeinen Formel R²-X, in der R² mit Ausnahme von Wasserstoff die in den Einzelverbindungen genannten Bedeutungen hat, nicht jedoch funktionelle Gruppen enthält, die die nachfolgenden Umsetzungen stören würden, und X die oben genannten Bedeutungen hat, alkyliert zu einem Produkt der allgemeinen Formel IV, 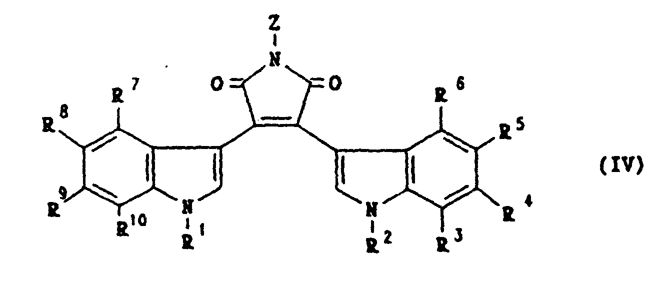 wonach man die substituierte Imidgruppe in die unsubstituierte Imidgruppe der Verbindung der allgemeinen Formel I überführt,
oder
B) ein Produkt der allgemeinen Formel V 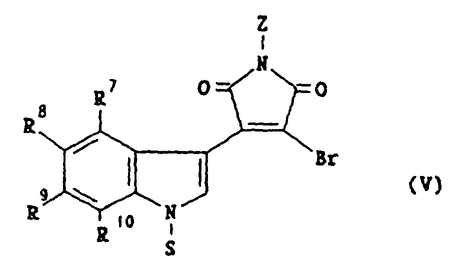 bei der S und Z eine geeignete abspaltbare Schutzgruppe bedeuten, mit einem Indolgrignardreagenz der allgemeinen Formel (B) 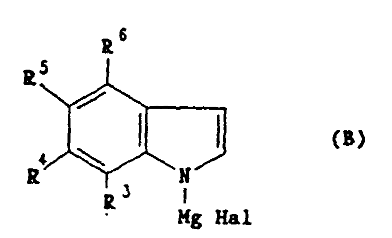 in welcher R³, R⁴, R⁵ und R⁶ die in den Einzelverbindungen genannte Bedeutung haben, nicht jedoch Hydroxy oder Acyloxy sind, umsetzt und gegebenenfalls anschließend mit einem Alkylierungsmittel der allgemeinen Formel R²-X, in der R² mit Ausnahme von Wasserstoff die in den Einzelverbindungen genannten Bedeutungen hat, nicht jedoch funktionelle Gruppen enthält, die die nachfolgenden Umsetzungen stören würden, und X die oben genannte Bedeutung hat, alkyliert zu einem Produkt der allgemeinen Formel VI 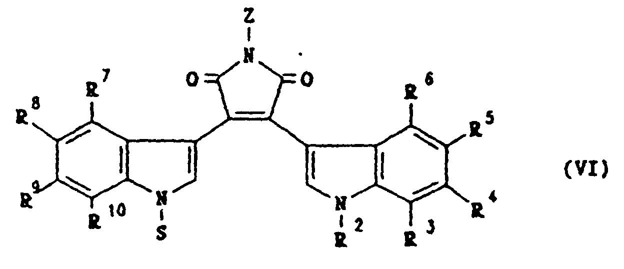 wonach man die geschützte Indolgruppe in die freie Indolgruppe und die substituierte Imidgruppe in die unsubstituierte Imidgruppe der Verbindung der allgemeinen Formel I überführt, wobei R¹ für Wasserstoff steht, und R² bis R¹⁰ die in den Einzelverbindungen genannte Bedeutung haben,
oder
C) ein Dibrommaleinimid der allgemeinen Formel II 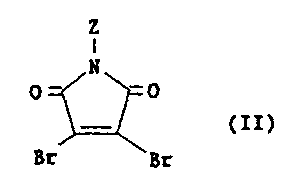 in welcher Z eine geeignete abspaltbare Schutzgruppe ist, mit einem Überschuß an Indolgrignardreagenz der allgemeinen Formel (A) 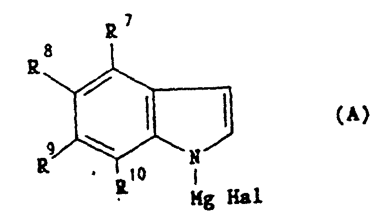 in welcher R⁷, R⁸, R⁹ und R¹⁰ die in den Einzelverbindungen genannte Bedeutung haben, nicht jedoch Hydroxy oder Acyloxy sind, umsetzt und das erhaltene Produkt der allgemeinen Formel VII 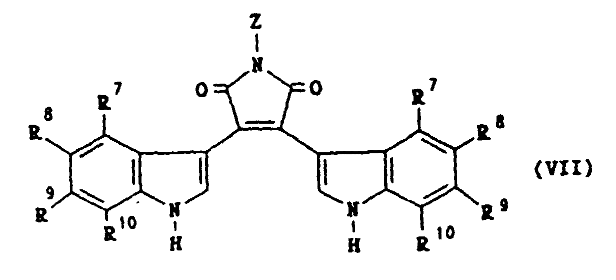 gegebenenfalls am Indolstickstoff mit einem Alkylierungsmittel R¹-X, wobei R¹ mit Ausnahme von Wasserstoff die in den Einzelverbindungen genannten Bedeutungen hat, nicht jedoch funktionelle Gruppen enthält, die die nachfolgenden Umsetzungen stören würden, und X für eine leicht austretende Gruppe wie Chlor oder Brom steht, alkyliert, wobei ein Produkt der allgemeinen Formel VIII 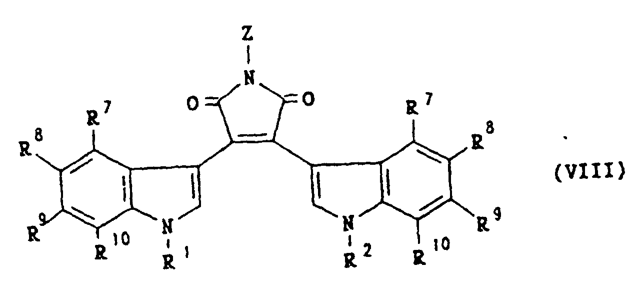 erhalten wird, bei der R¹ verschieden von Wasserstoff und R² gleich Wasserstoff und R⁷ bis R¹⁰ eine der in den Einzelverbindungen genannten Bedeutungen besitzen, nicht jedoch für Hydroxy oder Acyloxy stehen.

3. Arzneimittel enthaltend Bis-(1H-indol-3-yl)maleinimid-Derivate gemäß Anspruch 1.

4. Verwendung von Verbindungen gemäß Anspruch 1 zur Herstellung von Arzneimitteln zur Behandlung von Herz- und Gefäßkrankheiten wie Thrombosen, Arteriosklerose, Hypertension, von Entzündungsprozessen, Allergien, Krebs und bestimmten degenerativen Schäden des Zentralnervensystems sowie von Krankheiten des Immunsystems und viralen Erkrankungen.

## Claims

1. Bis-(1H-indol-3-yl)maleimide derivatives of the general formula I 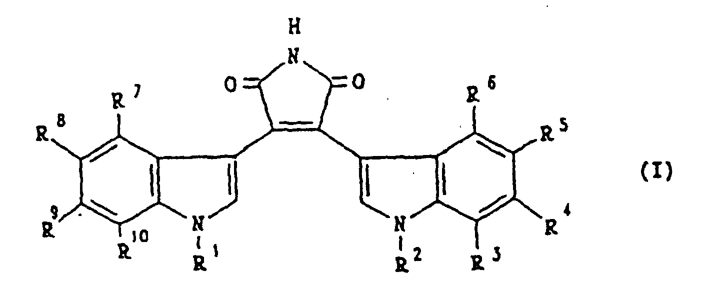 that is to say:
2-(1H-indol-3-yl)-3-(1-methyl-1H-indol-3-yl)maleimide
2-(1H-indol-3-yl)-3-[1-(3-dimethylaminopropyl)-1H-indol-3-yl]-maleimide
2-(5-hydroxy-1H-indol-3-yl)-3-(1H-indol-3-yl)maleimide
2-(5-benzyloxy-1H-indol-3-yl)-3-(1H-indol-3-yl)maleimide
2,3-bis(5-methoxy-1H-indol-3-yl)maleimide
2-(1H-indol-3-yl)-3-(5,6-methylenedioxy-1H-indol-3-yl)maleimide
2-(1H-indol-3-yl)-3-(5,6-dimethoxy-1H-indol-3-yl)maleimide
2,3-bis-(5,6-methylenedioxy-1H-indol-3-yl)maleimide
2-(1H-indol-3-yl)-3-(5-methoxy-1H-indol-3-yl)maleimide
2-(5-benzyloxy-1H-indol-3-yl)-3-[1-(3-dimethylaminopropyl)-1H-indol-3-yl]maleimide
2-[5-benzyloxy-1-(3-dimethylaminopropyl)-1H-indol-3-yl]-3-(1H-indol-3-yl)maleimide
2-(5-methoxy-1H-indol-3-yl)-3-[5-methoxy-1-(3-dimethylaminopropyl)-1H-indol-3-yl]maleimide
(±)-2-(1H-indol-3-yl)-3-[1-(3-dimethylamino-2-methoxypropyl)-1H-indol-3-yl]maleimide
2-(1H-indol-3-yl)-3-[5-(3-dimethylaminopropyloxy)-1H-indol-3-yl]maleimide
2,3-bis-(5-fluoro-1H-indol-3-yl)maleimide
2-(5-fluoro-1H-indol-3-yl)-3-[5-fluoro-1-(3-dimethylaminopropyl)-1H-indol-3-yl]maleimide
2,3-bis-(5-benzyloxy-1H-indol-3-yl)maleimide
2-(5-benzyloxy-1H-indol-3-yl)-3-[5-benzyloxy-1-(3-dimethylaminopropyl-1H-indol-3-yl]maleimide
2-[5-benzyloxy-1-(3-dimethylaminopropyl)-1H-indol-3-yl]-3-(1-methyl-1H-indol-3-yl)maleimide
2,3-bis-(5-chloro-1H-indol-3-yl)maleimide
2-(5-chloro-1H-indol-3-yl)-3-[5-chloro-1-(3-dimethylaminopropyl)-1H-indol-3-yl]maleimide
2,3-bis-(5-methyl-1H-indol-3-yl)maleimide
2-(5-methyl-1H-indol-3-yl)-3-[5-methyl-1-(3-dimethylaminopropyl)-1H-indol-3-yl]maleimide
2,3-bis-(5-bromo-1H-indol-3-yl)maleimide
2-(5-bromo-1H-indol-3-yl)-3-[5-bromo-1-(3-dimethylaminopropyl)-1H-indol-3-yl]maleimide
2-(1H-indol-3-yl)-3-[1-(2-dimethylaminoethyl)-1H-indol-3-yl]-maleimide;
2-(1H-indol-3-yl)-3-[1-(3-(1-piperidino)propyl)-1H-indol-3-yl]-maleimide;
2-(1H-indol-3-yl)-3-[1-(3-(4-morpholino)propyl-1H-indol-3-yl]-maleimide;
2-[1-(3-diethylaminopropyl)-1H-indol-3-yl]-3-(1H-indol-3-yl)-maleimide;
(±)-2-[1-(3-diethylamino-2-hydroxypropyl)-1H-indol-3-yl]-3-(1H-indol-3-yl)-maleimide;
(+)-2-[1-(3-diethylamino-2-hydroxypropyl)-1H-indol-3-yl]-3-(1H-indol-3-yl)-maleimide;
(-)-2-[1-(3-diethylamino-2-hydroxypropyl)-1H-indol-3-yl]-3-(1H-indol-3-yl)-maleimide;
2-(5-methoxy-1H-indol-3-yl)-3-[1-(3-dimethylaminopropyl)-1H-indol-3-yl]-maleimide;
2-(1H-indol-3-yl)-3-[5-methoxy-1-(3-dimethylaminopropyl)-1H-indol-3-yl]-maleimide;
(±)-2-[1-(2,3-dihydroxypropyl)-1H-indol-3-yl]-3-(1H-indol-3-yl)-maleimide;
(+)-2-[1-(2,3-dihydroxypropyl)-1H-indol-3-yl]-3-(1H-indol-3-yl)-maleimide;
(-)-2-[1-(2,3-dihydroxypropyl)-1H-indol-3-yl]-3-(1H-indol-3-yl]-maleimide;
2-(5-methoxy-1H-indol-3-yl)-3-[1-(3-(4-morpholino)propyl)-1H-indol-3-yl]-maleimide;
2-(1H-indol-3-yl)-3-[5-methoxy-1-(3-(4-morpholino)propyl)-1H-indol-3-yl]-maleimide;
(±)-2-[1-(3-ethoxy-2-hydroxypropyl)-1H-indol-3-yl]-3-(1H-indol-3-yl)-maleimide;
(+)-2-[1-(3-ethoxy-2-hydroxypropyl)-1H-indol-3-yl]-3-(1H-indol-3-yl)-maleimide;
(-)-2-[1-(3-ethoxy-2-hydroxypropyl)-1H-indol-3-yl]-3-(1H-indol-3-yl)-maleimide;
(±)-2-[1-(2-hydroxy-3-(1-piperidino)-propyl)-1H-indol-3-yl]-3-(1H-indol-3-yl)-maleimide;
(+)-2-[1-(2-hydroxy-3-(1-piperidino)-propyl)-1H-indol-3-yl)-3-(1H-indol-3-yl)-maleimide;
(-)-2-[1-(2-hydroxy-3-(1-piperidio)-propyl)-1H-indol-3-yl]-3-(1H-indol-3-yl)-maleimide;
(±)-2-[1-(3-diethylamino-2-hydroxypropyl)-5-methoxy-1H-indol-3-yl]-3-(1H-indol-3-yl)-maleimide;
(+)-2-[1-(3-diethylamino-2-hydroxypropyl)-5-methoxy-1H-indol-3-yl]-3-(1H-indol-3-yl)-maleimide;
(-)-2-[1-(3-diethylamino-2-hydroxypropyl)-5-methoxy-1H-indol-3-yl]-3-(1H-indol-3-yl]-maleimide;
(±)-2-[1-(3 -diethylamino-2-hydroxypropyl)-1H-indol-3-yl]-3-(5-methoxy-1H-indol-3-yl)-maleimide;
(+)-2-[1-(3-diethylamino-2-hydroxypropyl)-1H-indol-3-yl]-3-(5-methoxy-1H-indol-3-yl)-maleimide;
(-)-2-[1-(3-diethylamino-2-hydroxypropyl)-1H-indol-3-yl]-3-(5-methoxy-1H-indol-3-yl)-maleimide;
2-(1H-indol-3-yl)-3-[5-methoxy-1-(3-(1-piperidino)propyl)-1H-indol-3-yl]-maleimide;
2-(5-methoxy-1H-indol-3-yl)-3-[1-(3-(1-piperidino)propyl)-1H-indol-3-yl]-maleimide;
2-[1-(3-diethylaminopropyl)-5-methoxy-lH-indol-3-yl]-3-(5,6-dimethoxy-1-methyl-1H-indol-3-yl)-maleimide;
2-[1-(3-diethylaminopropyl)-5-methoxy-1H-indol-3-yl]-3-(1H-indol-3-yl)-maleimide;
2-[1-(3-diethylaminopropyl)-1H-indol-3-yl)-3-(5-methoxy-1H-indol-3-yl)-maleimide;
2-[1-(3-diethylaminopropyl)-5-methoxy-1H-indol-3-yl]-3-(6-methoxy-1H-indol-3-yl)-maleimide;
2-[5-fluoro-1-(3-dimethylaminopropyl)-1H-indol-3-yl]-3-(1H-indol-3-yl)-maleimide;
2-(5-fluoro-1H-indol-3-yl)-3-[1-(3-dimethylaminopropyl)-1H-indol-3-yl]-maleimide;
2-[1-(3-diethylaminopropyl)-6-methoxy-1H-indol-3-yl]-3-(5-methoxy-1H-indol-3-yl)-maleimide;
2-(5-bromo-1H-indol-3-yl)-3-(1H-indol-3-yl)-maleimide;
2-(5-fluoro-1H-indol-3-yl)-3-(1H-indol-3-yl)-maleimide;
2-[1-(3-diethylaminopropyl)-5-fluoro-1H-indol-3-yl]-3-(1H-indol-3-yl)-maleimide;
(±)-2-[1-(2,3-dihydroxypropyl)-5-methoxy-1H-indol-3-yl]-3-(1H-indol-3-yl)-maleimide
and
(±)-2-[1-(3-ethoxy-2-hydroxypropyl)-5-methoxy-1H-indol-3-yl]-3-(1H-indol-3-yl)-maleimide.

2. Process for the preparation of compounds according to claim 1, **characterized in that** either
A) a dibromomaleimide of the general formula II 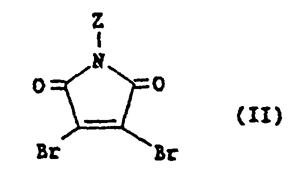 in which Z is a suitable protective group which can be split off, is reacted with an indole Grignard reagent of the general formula A, 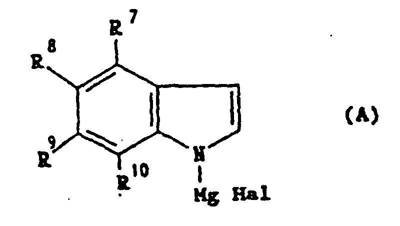 in which R⁷, R⁸, R⁹ and R¹⁰ have the meaning mentioned in the individual compounds, but are not hydroxyl or acyloxy, by methods which are known per se, and the resulting product of the general formula III 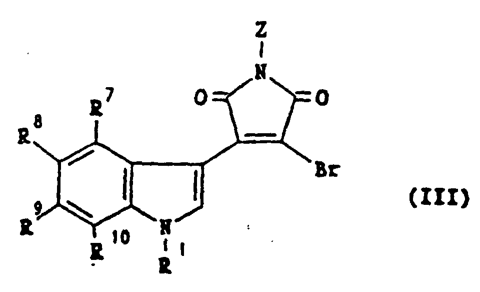 in which R¹ denotes hydrogen, is optionally alkylated in a manner known per se on the indole nitrogen with an alkylating agent R¹-X, wherein R¹ has the meanings mentioned in the individual compounds, with the exception of hydrogen, but does not contain functional groups which would interfere with the subsequent reactions, and X represents a group which leaves readily, such as chlorine or bromine, a product of the general formula III in which R¹ is other than hydrogen being obtained, and the product III is then reacted with an indole Grignard reagent of the general formula (B) 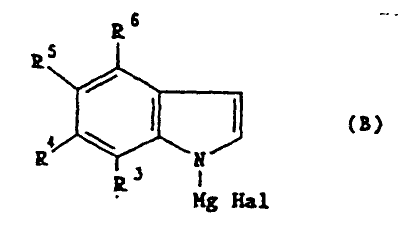 in which R³, R⁴, R⁵ and R⁶ have the meaning mentioned in the individual compounds, but are not hydroxyl or acyloxy, and is optionally then alkylated with an alkylating agent of the general formula R²-X, in which R² has the meanings mentioned in the individual compounds, with the exception of hydrogen, but does not contain functional groups which would interfere with the subsequent reactions, and X has the abovementioned meanings, to give a product of the general formula IV 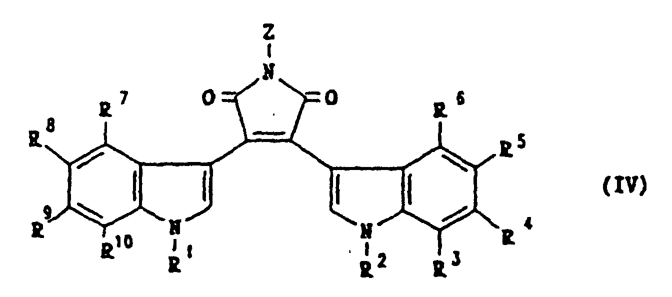 after which the substituted imide group is converted into the unsubstituted imide group of the compound of the general formula I,
or
B) a product of the general formula V 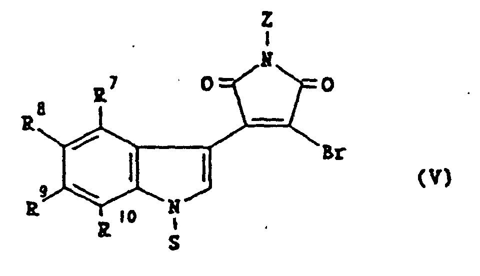 in which S and Z denote a suitable protective group which can be split off, is reacted with an indole Grignard reagent of the general formula (B) 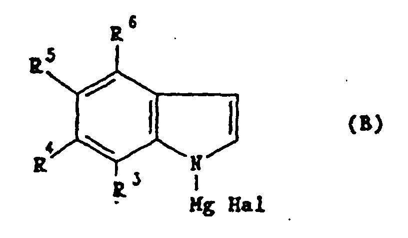 in which R³, R⁴, R⁵ and R⁶ have the meaning mentioned in the individual compounds, but are not hydroxyl or acyloxy, and optionally then alkylated with an alkylating agent of the general formula R²-X, in which R² has the meanings mentioned in the individual compounds, with the exception of hydrogen, but does not contain functional groups which would interfere with the subsequent reactions, and X has the abovementioned meaning, to give a product of the general formula VI 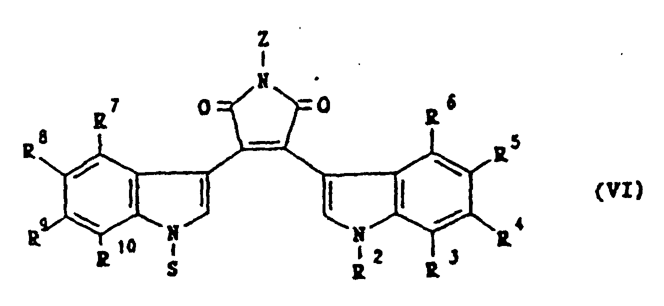 after which the protected indole group is converted into the free indole group and the substituted imide group is converted into the unsubstituted imide group of the compound of the general formula I, wherein R¹ represents hydrogen and R² to R¹⁰ have the meaning mentioned in the individual compounds,
or
C) a dibromomaleimide of the general formula II 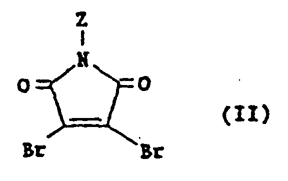 in which Z is a suitable protective group which can be split off, is reacted with an excess of indole Grignard reagent of the general formula (A) 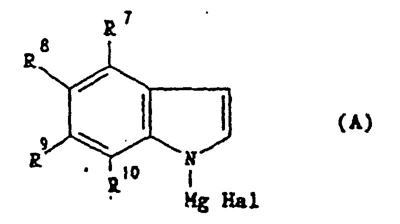 in which R⁷, R⁸, R⁹ and R¹⁰ have the meaning mentioned in the individual compounds, but are not hydroxyl or acyloxy, and the resulting product of the general formula VII 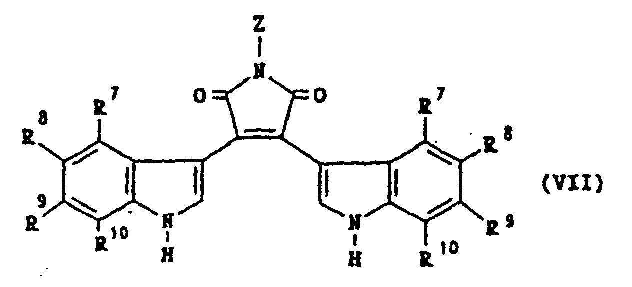 is optionally alkylated on the indole nitrogen with an alkylating agent R¹-X, wherein R¹ has the meanings mentioned in the individual compounds, with the exception of hydrogen, but does not contain functional groups which would interfere with the subsequent reactions, and X represents a group which leaves readily, such as chlorine or bromine, a product of the general formula VIII 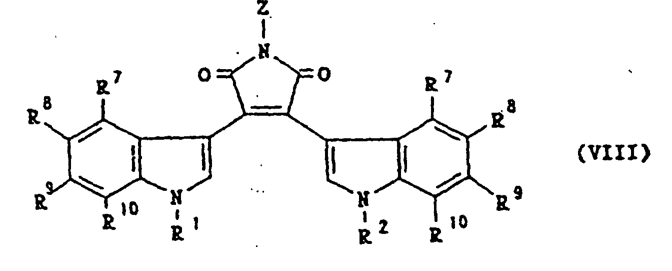 being obtained, in which R¹ is other than hydrogen and R² is hydrogen and R⁷ to R¹⁰ have one of the meanings mentioned in the individual compounds, but do not represent hydroxyl or acyloxy.

3. Medicaments comprising bis-(1H-indol-3-yl)maleimide derivatives according to claim 1.

4. Use of compounds according to claim 1 for the preparation of medicaments for treatment of cardiovascular diseases, such as thromboses, arteriosclerosis or hypertension, of inflammation processes, allergies, cancer and certain degenerative damage to the central nervous system, and of diseases of the immune system and viral diseases.

## Revendications

1. Dérivés de bis-(1H-indol-3-yl) maléinimide, de formule générale I, 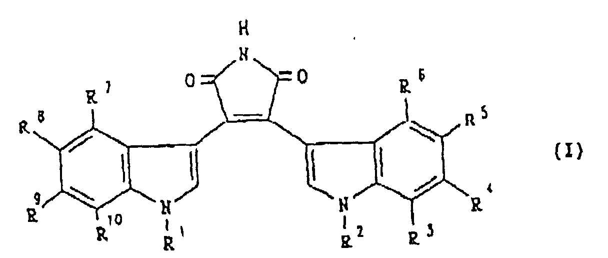 à savoir:
2-(1H-indol-3-yl)-3-(1-méthyl-1H-indol-3-yl)-maléinimide,
2-(1H-indol-3-yl)-3-[1-(3-diméthylaminopropyl)-1H-indol-3-yl]-maléinimide,
2-(5-hydroxy-1H-indol-3-yl)-3-1H-indol-3-yl)-maléinimide,
2-(5-benzyloxy-1H-indol-3-yl)-3-(1H-indol-3-yl)-maléinimide,
2,3-bis-(5-methoxy-1H-indol-3-yl)-maléinimide,
2-(1H-indol-3-yl)-3-(5,6-méthylènedioxy-1H-indol-3-yl)-maléinimide,
2-(1H-indol-3-yl)-3-(5,6-diméthoxy-1H-indol-3-yl)-maléinimide,
2,3-bis-(5,6-méthylènedioxy-1H-indol-3-yl)-maléinimide,
2-(1H-indol-3-yl)-3-(5-méthoxy-1H-indol-3-yl)-maléinimide,
2-(5-benzyloxy-1H-indol-3-yl)-3-[1-(3-diméthylaminopropyl)-1H-indol-3-yl]-maléinimide,
2-[5-benzyloxy-1-(3-diméthylaminopropyl)-1H-indol-3-yl]-3-(1H-indol-3-yl)-maléinimide,
2-(5-méthoxy-1H-indol-3-yl)-3-[5-méthoxy-1-(3-diméthylaminopropyl)-1H-indol-3-yl]-maléinimide,
(±)-2-(1H-indol-3-yl)-3-[1-(3-diméthylamino-2-méthoxypropyl)-1H-indol-3-yl]-maléinimide,
2-(1H-indol-3-yl)-3-[5-(3-diméthylaminopropyloxy)-1H-indol-3-yl]-maléinimide,
2,3-bis-(5-fluor-1H-indol-3-yl)-maléinimide,
2-(5-fluor-1H-indol-3-yl)-3-[5-fluor-1-(3-diméthylaminopropyl)-1H-indol-3-yl)-maléinimide,
2,3-bis-(5-benzyloxy-1H-indol-3-yl)-maléinimide,
2-(5-benzyloxy-1H-indol-3-yl)-3-[5-benzyloxy-1-(3-diméthylaminopropyl-1H-indol-3-yl]-maléinimide,
2-[5-benzyloxy-1-(3-diméthylaminopropyl)-1H-indol-3-yl]-3-[1-méthyl-1H-indol-3-yl)-maléinimide,
2,3-bis-(5-chloro-1H-indol-3-yl)-maléinimide,
2-(5-chloro-1H-indol-3-yl)-3-[5-chloro-1-(3-diméthylaminopropyl)-1H-indol-3-yl]-maléinimide,
2,3-bis-(5-méthyl-1H-indol-3-yl)-maléinimide,
2-(5-méthyl-1H-indol-3-yl)-3-[5-chloro-1-(3-diméthylaminopropyl)-1H-indol-3-yl)-maléinimide,
2,3-bis-(bromo-1H-indol-3-yl)-maléinimide,
2-[bromo-1H-indol-3-yl)-3-(5-bromo-1-(3-diméthylaminopropyl)-1H-indol-3-yl]-maléinimide,
2-(1H-indol-3-yl)-3-[1-(2-diméthylaminoéthyl)-1H-indol-3-yl)-maléinimide,
2-(1H-indol-3-yl)-3-[1-(3-(1-pipéridino)-propyl)-1H-indol-3-yl]-maléinimide,
2-(1H-indol-3-yl)-3-[1-(3-(4-morpholino)-propyl)-1H-indol-3-yl]-maléinimide,
2-[1-(3-diéthylaminopropyl)-1H-indol-3-yl]-3-(1H-indol-3-yl)-maléinimide,
(±)-2-[1-(3-diéthylamino-2-hydroxypropyl)-1H-indol-3-yl]-3-(1H-indol-3-yl)-maléinimide,
(+)-2-[1-(3-diéthylamino-2-hydroxypropyl)-1H-indol-3-yl]-3-(1H-indol-3-yl)-maléinimide,
(-)-2-[1-(3-diéthylamino-2-hydroxypropyl)-1H-indol-3-yl]-3-(1H-indol-3-yl)-maléinimide,
2-(5-méthoxy-1H-indol-3-yl)-3-[1-(3-diméthylaminopropyl)-1H-indol-3-yl]-maléinimide,
2-(1H-indol-3-yl)-3-[5-méthoxy-1-(3-diméthylaminopropyl)-lH-indol-3-yl]-maléinimide,
(±)-2-[1-(2,3-dihydroxypropyl)-1H-indol-3-yl]-3-(1H-indol-3-yl)-maléinimide,
(+)-2-[1-(2,3-dihydroxypropyl)-1H-indol-3-yl]-3-(1H-indol-3-yl)-maléinimide,
(-)-2-[1-(2,3-dihydroxypropyl)-1H-indol-3-yl]-3-(1H-indol-3-yl)-maléinimide,
2-(5-méthoxy-1H-indol-3-yl)-3-[1-(3-(4-morpholino)-propyl]-1H-indol-3-yl]-maléinimide,
2-(1H-indol-3-yl)-3-[5-méthoxy-1-(3-(4-morpholino)-propyl]-1H-indol-3-yl]-maléinimide,
(±)-2-[1-(3-éthoxy-2-hydroxypropyl)-1H-indol-3-yl]-3-(1H-indol-3-yl)-maléinimide,
(+)-2-[1-(3-éthoxy-2-hydroxypropyl)-1H-indol-3-yl]-3-(1H-indol-3-yl)-maléinimide,
(-)-2-[1-(3-éthoxy-2-hydroxypropyl)-1H-indol-3-yl]-3-(1H-indol-3-yl)-maléinimide,
(±)-2-[1-(2-hydroxy-3-(1-pipéridino)-propyl)-1H-indol-3-yl]-3-(1H-indol-3-yl)-maléinimide,
(+)-2-[1-(2-hydroxy-3-(1-pipéridino)-propyl)-1H-indol-3-yl]-3-(1H-indol-3-yl)-maléinimide,
(-)-2-[1-(2-hydroxy-3-(1-pipéridino)-propyl)-1H-indol-3-yl]-3-(1H-indol-3-yl)-maléinimide,
(±)-2-[1-(3-diéthylamino-2-hydroxypropyl)-5-méthoxy-1H-indol-3-yl]-3-(1H-indol.3-yl)-maléinimide,
(+)-2-[1-(3-diéthylamino-2-hydroxypropyl)-5-méthoxy-1H-indol-3-yl]-3-(1H-indol-3-yl)-maléinimide,
(-)-2-[1-(3-diéthylamino-2-hydroxypropyl)-5-méthoxy-1H-indol-3-yl]-3-(1H-indol-3-yl)-maléinimide,
(±)-2-[1-(3-diéthylamino-2-hydroxypropyl)-1H-indol-3-yl]-3-(5-méthoxy-1H-indol-3-yl)-maléinimide,
(+)-2-[1-(3-diéthylamino-2-hydroxypropyl)-1H-indol-3-yl]-3-(5-méthoxy-1H-indol-3-yl)-maléinimide,
(-)-2-[1-(3-diéthylamino-2-hydroxypropyl)-1H-indol-3-yl]-3-(5-méthoxy-1H-indol-3-yl)-maléinimide,
2-(1H-indol-3-yl)-3-[5-méthoxy-1-(3-(1-pipéridino)-propyl)-1H-indol-3-yl]-maléinimide,
2-(5-méthoxy-1H-indol-3-yl)-3-[1-(3-(1-pipéridino)-propyl)-1H-indol-3-yl]-maléinimide,
2-[1-(3-diéthylaminopropyl)-5-méthoxy-1H-indol-3-yl]-3-(5,6-diméthoxy-1-méthyl-1H-indol-3-yl)-maléinimide,
2-[1-(3-diéthylaminopropyl)-5-méthoxy-1H-indol-3-yl]-3-(1H-indol-3-yl)-maléinimide,
2-[1-(3-diéthylaminopropyl)-1H-indol-3-yl]-3-(5-méthoxy-1H-indol-3-yl)-maléinimide,
2-[1-(3-diéthylaminopropyl)-5-méthoxy-1H-indol-3-yl]-3-(6-méthoxy-1H-indol-3-yl)-maléinimide,
2-[5-fluoro-1-(3-diméthylaminopropyl)-1H-indol-3-yl]-3-(1H-indol-3-yl)-maléinimide,
2-(5-fluoro-1H-indol-3-yl)-3-[1-(3-diméthylaminopropyl)-1H-indol-3-yl]-maléinimide,
2-[1-(3-diméthylaminopropyl)-6-méthoxy-1H-indol-3-yl]-3-(5-méthoxy-1H-indol-3-yl)-maléinimide,
2-(5-bromo-1H-indol-3-yl)-3-(1H-indol-3-yl)-maléinimide,
2-(5-fluoro-1H-indol-3-yl)-3-(1H-indol-3-yl)-maléinimide,
2-[1-(3-diéthylaminopropyl)-5-fluoro-1H-indol-3-yl]-3-(1H-indol-3-yl)-maléinimide,
(±)-2-[1-(2,3-dihydroxypropyl)-5-méthoxy-1H-indol-3-yl]-3-(1H-indol-3-yl)-maléinimide,
(±)-2-[1-(3-éthoxy-2-hydroxypropyl)-5-méthoxy-1H-indol-3-y]-3-(1H-indol-3-yl)-maléinimide,

2. Procédé pour la préparation de composés selon la revendication 1, **caractérisé en ce que** soit :
A) on fait réagir un dibromomaléinimide de formule générale II 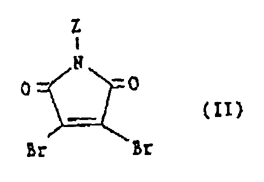 dans laquelle Z est un groupe de protection facilement éliminé approprié, avec un réactif de Grignard à base d'indol, de formule générale A, 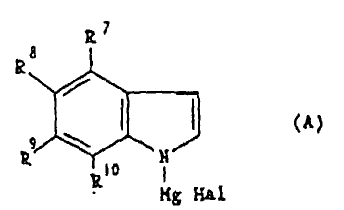 dans laquelle R⁷, R⁸, R⁹ et R¹⁰ ont les significations données dans les composés spécifiés, à l'exclusion des significations hydroxy et acyloxy, selon des méthodes connues en soi, et **en ce que** le produit obtenu de formule générale III, 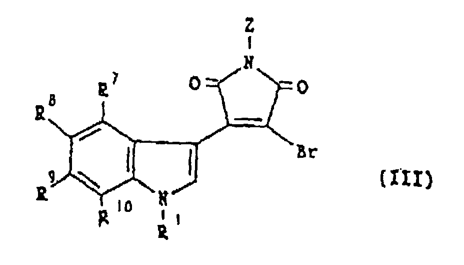 dans laquelle R¹ est un atome d'hydrogène, est alkylé de manière connue en soi, le cas échéant sur l'azote de l'indol par un réactif d'alkylation de formule:
R¹-X,
dans laquelle R¹ a la signification donnée dans les composés spécifiés, sans cependant consister en un atome d'hydrogène, et ne contient en outre pas de groupes fonctionnels qui gêneraient les réactions subséquentes, et X représente un groupe partant, tel que le chlore ou le brome, ce qui conduit à obtenir un produit de formule générale III, dans laquelle R¹ est autre qu'un atome d'hydrogène, et le produit de formule III est ensuite amené à réagir avec un réactif de Grignard à base d'indol de formule (B), 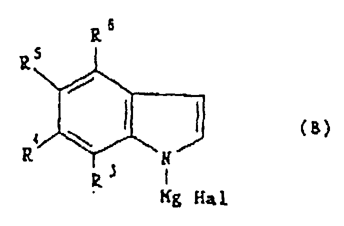 dans laquelle R³, R⁴, R⁵ et R⁶ ont les significations données dans les composés spécifiés, à l'exclusion des significations hydroxy et acyloxy, et puis ensuite on alkyle, le cas échéant avec un réactif d'alkylation de formule:
R²-X,
dans laquelle R² a la signification donnée dans les composés spécifiés, sans cependant consister en un atome d'hydrogène, et ne contient en outre pas de groupes fonctionnels qui gêneraient les réactions subséquentes, et X a les significations données ci-dessus, et obtient ainsi un produit de formule générale IV, 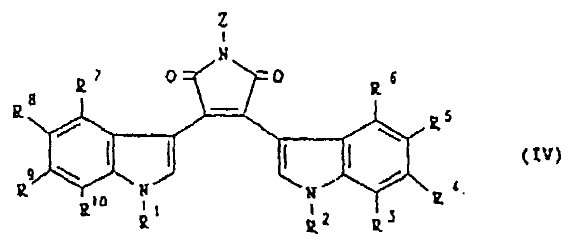 ce après quoi on transforme le groupe imide substitué en le groupe imide non substitué du composé de formule générale I,
soit,
B) on fait réagir un produit de formule générale V : 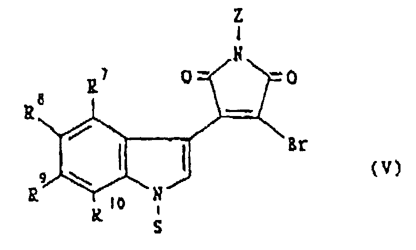 dans laquelle S et Z sont un groupe de protection facilement éliminé approprié, avec un réactif de Grignard à base d'indol, de formule générale (B) 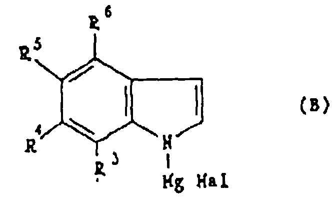 dans laquelle R³, R⁴, R⁵ et R⁶ ont les significations données dans les composés spécifiés, à l'exclusion des significations hydroxy et acyloxy,
et puis ensuite on alkyle le cas échéant avec un réactif d'alkylation de formule :
R²-X,
dans laquelle R² a la signification donnée dans les composés spécifiés, sans cependant consister en un atome d'hydrogène, et ne contient en outre pas de groupes fonctionnels qui gêneraient les réactions subséquentes, et X a les significations données ci-dessus. pour ainsi obtenir un produit de formule générale VI, 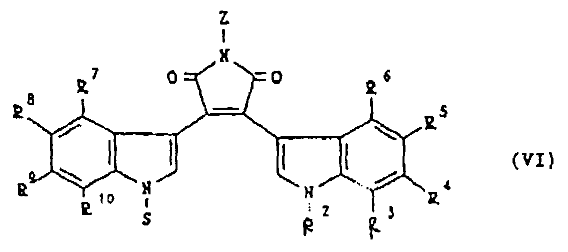 ce après quoi on transforme le groupe indol protégé en un groupe indol libre et le groupe imide substitué en le groupe imide non substitué du composé de formule générale I, dans laquelle R¹ consiste en un atome d'hydrogène et R² à R¹⁰ ont les significations données dans les composés spécifiés ;
soit,
C) on fait réagir un dibromomaléinimide, de formule générale II : 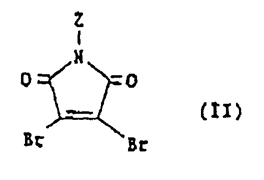 dans laquelle Z est un groupe de protection facilement éliminé approprié, avec un excès de réactif de Grignard à base d'indol de formule générale (A) 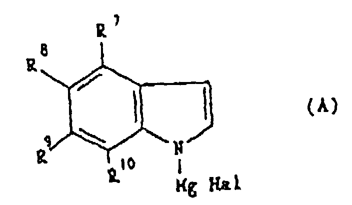 dans laquelle R⁷, R⁸, R⁹ et R¹⁰ ont les significations données dans les composés spécifiés, à l'exclusion des significations hydroxy et acyloxy, et **en ce que** le produit obtenu, de formule générale VII. 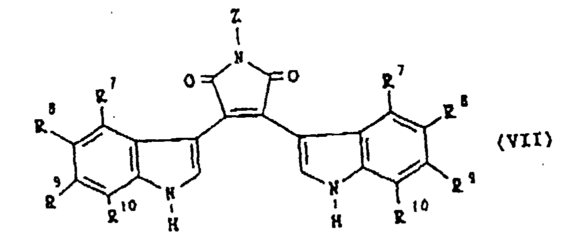 est ensuite alkylé, le cas échéant, sur l'azote de l'indol par un réactif d'alkylation de formule :
R¹-X,
dans laquelle R¹ a la signification donnée dans les composés spécifiés, sans cependant consister en un atome d'hydrogène, et ne contient en outre pas de groupes fonctionnels qui gêneraient les réactions subséquentes, et X représente un groupe partant, tel que le chlore ou le brome, obtenant ainsi un produit de formule générale VIII, 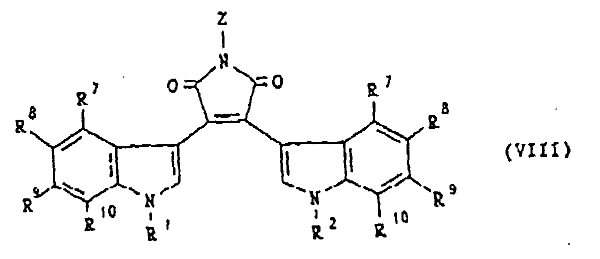 dans laquelle R¹ est autre qu'un atome d'hydrogène, R² est l'hydrogène et R⁷à R¹⁰ ont les significations données pour les composés spécifiés à l'exception cependant des significations hydroxy et acyloxy.

3. Médicament contenant des dérivés de bis-(1H-indol-3-yl)-maléinimide selon la revendication 1.

4. Utilisation de composés selon la revendication 1 pour la préparation de médicaments pour le traitement de maladies cardiovasculaires telles que thromboses, artériosclérose, hypertension, processus inflammatoires, allergies, cancers et certaines lésions du système central nerveux ainsi que de maladies du système immunitaire et de maladies virales.
